(19) Europäisches Patentamt
European Patent Office
Office européen des brevets

(11) EP 0 834 304 B1

(12) EUROPÄISCHE PATENTSCHRIFT

(45) Veröffentlichungstag und Bekanntmachung des Hinweises auf die Patenterteilung:
**26.01.2005 Patentblatt 2005/04**

(51) Int Cl.[7]: **A61K 7/42**, A61K 7/00, C07D 251/24, A61K 7/06

(21) Anmeldenummer: **97810641.7**

(22) Anmeldetag: **09.09.1997**

(54) **Verwendung liposomogener UV-Absorber zum Schutz des Haars**

Use of liposomogenic UV absorbers to protect hair

Utilisation des absorbeurs UV liposomogènes pour la protection des cheveux

(84) Benannte Vertragsstaaten:
**AT BE CH DE DK ES FI FR GB IE IT LI NL SE**

(30) Priorität: **17.09.1996 CH 227396**

(43) Veröffentlichungstag der Anmeldung:
**08.04.1998 Patentblatt 1998/15**

(73) Patentinhaber: **Ciba Specialty Chemicals Holding Inc.**
**4057 Basel (CH)**

(72) Erfinder:
- **Luther, Helmut**
**79639 Grenzach-Wyhlen (DE)**
- **Hüglin, Dietmar**
**D-79591 Eimeldingen (DE)**
- **Hochberg, Robert**
**79100 Freiburg (DE)**

(56) Entgegenhaltungen:
**EP-A- 0 743 309**
**WO-A-95/00111**
**WO-A-96/03968**
**WO-A-96/29302**
**GB-A- 2 293 823**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 834 304 B1

## Beschreibung

Die vorliegende Erfindung betrifft die Verwendung von liposomogenen UV-Absorbern zum Schützen von menschlichem Haar vor der schädigenden Einwirkung von UV-Strahlung.

Wird Human-Haar über längere Zeit dem Sonnenlicht ausgesetzt, können verschiedene Schädigungen auftreten. Dunkles Haar erfährt nach einiger Zeit einen rötlichen Farbton, blondes Haar wird gelblich. Die Oberfläche des Haars wird rauher und gleichzeitig trockener. Ausserdem verliert es mit der Zeit seinen Glanz.

Durch die Verwendung von UV-Absorbern können Haare wirkungsvoll vor schädlichen Sonnenstrahlen geschützt werden. Leider besitzen die bisher verwendeten UV-Absorber ein auf Human-Haar unzureichendes Aufziehvermögen, d.h. sie lassen sich leicht herauswaschen und haben daher nur eine kurzzeitige Wirkung.

Ueberraschenderweise wurde nun gefunden, dass Verbindungen, die einen UV-Chromophor mit einer Absorption im Bereich von 285 bis 400 nm und spezielle chemische Strukturelemente enthalten, die diese Verbindungen zur Selbstorganisation in bimolekularen Schichten befähigen, eine sehr gute Substantivität gegenüber Human-Haar aufweisen und gleichzeitig einen wirkungsvollen UV-Schutz für das Haar bilden.

Gegenstand der vorliegenden Erfindung ist daher die Verwendung liposomogener UV-Absorber, die eine hydrophile Kopfgruppe (=Z), einen Abstandhalter (=W), ein UV-Chromophor (Q) mit einer Absorption im Bereich von 285 bis 400 nm und mindestens eine hydrophobe Schwanzgruppe (=A) aufweisen zum Schützen von menschlichem Haar vor der schädigenden Einwirkung von UV-Strahlung.

Die liposomogenen UV-Absorber entsprechen der Formel

(1) 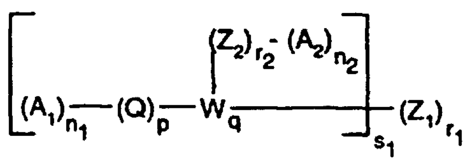 ,

worin

$A_1$ und $A_2$, unabhängig voneinander, einen hydrophoben Rest;

Q einen UV-Chromophor;

W einen organischen Rest;

$Z_1$ und $Z_2$, unabhängig voneinander, einen hydrophilen Rest;

$n_1$ und $n_2$, unabhängig voneinander, eine Zahl von 0 bis 4, wobei $n_1$=$n_2$=0 nicht mitumfasst ist;

p 1 oder 2;

q eine Zahl von 0 bis 3;

$r_1$ 1 oder 2;

$r_2$ 0 oder 1; und

$s_1$ eine Zahl von 1 bis 3;

bedeuten.

Diese Verbindungen stellen synthetische amphiphile Verbindungen dar, die in der Lage sind, sich selbst zu organisieren, d.h. sie bilden in Wasser spontan eine zweidimensionale Doppelschicht aus.

Die hydrophoben Reste $A_1$ und $A_2$ stellen einen Alkyl-, Alkoxy-, Acyl- oder Alkylaminorest dar, wobei die Ketten mindestens 8 Kohlenstoffatome aufweisen. Als Alkoxyreste stellen $A_1$ und $A_2$ vorteilhafterweise den Rest eines ungesättigten oder vorzugsweise gesättigten aliphatischen Monoalkohols mit 8 bis 22 Kohlenstoffatomen dar. Der Kohlenwasserstoffrest kann verzweigt oder vorzugsweise geradkettig sein. Vorzugsweise bedeuten $A_1$ und $A_2$ einen Alkyl- oder Alkenylrest mit 10 bis 14 Kohlenstoffatomen.

Als aliphatische gesättigte Monoalkohole können natürliche Alkohole, wie z.B. Laurylalkohol, Myristylalkohol,

Cetylalkohol oder Stearylalkohol, sowie synthetische Alkohole, wie z.B. Decylalkohol, $C_{10}$-$C_{13}$-Oxoalkohol, Tridecylalkohol, Isotridecylalkohol oder lineare primäre Alkohole (Alfole) mit 10 bis 22 Kohlenstoffatomen in Betracht kommen. Einige Vertreter dieser Alfole sind Alfol (10-14), Alfol (12-13) oder Alfol (16-18). ("Alfol" ist ein eingetragenes Warenzeichen).

Ungesättigte aliphatische Monoalkohole sind beispielsweise Dodecenylalkohol, Hexadecenylalkohol oder Oleylalkohol.

Die Alkoholreste können einzeln oder in Form von Gemischen, wie z.B. Mischungen von Alkyl- und/oder Alkenylgruppen, die sich von Soja-Fettsäuren, Palmkernfettsäuren oder Talg-Oelen ableiten, aus zwei oder mehreren Komponenten, vorzugsweise einzeln vorhanden sein.

Bedeuten die Reste $A_1$ und $A_2$ einen Alkylaminorest, so leitet sich dieser von primären oder vorzugsweise sekundären $C_{12}$-$C_{22}$-Fettaminen ab. Diese Amine lassen sich aus den entsprechenden Fettsäuren durch Dehydratisierung und anschliessender Dehydrierung gewinnen. Vorzugsweise bedeuten $A_1$ und $A_2$ als Alkylaminorest den Di-$C_{12}$-$C_{18}$-Alkylaminorest.

Als Acylrest kommt vorzugsweise $C_8$-$C_{22}$-Alkylcarbonyl in Betracht; wie z.B. Octyl-, Decyl-, Dodecyl-, Tridecyl-, Hexadecyl- oder Octadecylcarbonyl.

Vorzugsweise kommen als liposomogene UV-Absorber Verbindungen der Formel (1) in Betracht, bei denen $n_1$ und $n_2$ 1 oder 2 , oder vorzugsweise 2 bedeuten und die Reste $A_1$ und $A_2$ die gleiche Bedeutung haben.

Der UV-Chromophor Q leitet sich von an sich bekannten UV-Absorbern ab. Vorzugsweise kommen für die erfindungsgemässen liposomogenen UV- Chromophore Verbindungen mit Strukturelementen aus der Klasse der

($Q_1$) Zimtsäureester;
($Q_2$) Triazinderivate;
($Q_3$) Benzotriazole;
($Q_4$) Benzophenone;
($Q_5$) p-Aminobenzoesäurederivate; und
($Q_6$) Benzylidencampher

in Betracht.

Bevorzugte UV-Chromophore sind ($Q_1$) Zimtsäureester und ($Q_2$) Triazinderivate.

Bei den Zimtsäureestem $Q_1$ handelt es sich um Verbindungen der Formel

(2) 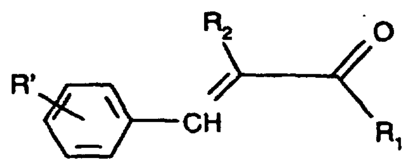

worin

R' Wasserstoff, $C_1$-$C_4$-Alkyl; oder $C_1$-$C_4$-Alkoxy;
$R_1$ $C_1$-$C_4$-Alkoxy; vorzugsweise Methoxy oder Ethoxy; und
$R_2$ Wasserstoff; $C_1$-$C_4$-Alkyl; oder -CN;

bedeuten.

Beispielhafte Verbindungen der Formel (2) sind der Zimtsäuremethyl- oder Zimtsäureethylester.

Bei den Triazinderivaten $Q_2$ handelt es sich z.B. um Hydroxyphenyl-s-triazine der Formel

(3)

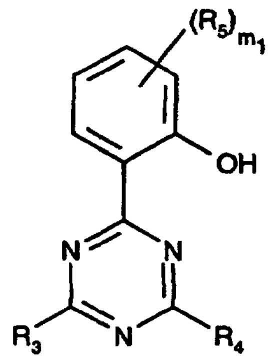

worin

R3 und R4, unabhängig voneinander, $C_1$-$C_5$-Alkyl; durch Hydroxy, $C_1$-$C_5$-Alkoxy, $C_1$-$C_5$-Alkylthio, Amino oder $C_1$-$C_5$-Mono- oder -Dialkylamino substituiertes $C_1$-$C_{18}$-Alkyl; nicht substituiertes Phenyl; oder durch Chlor, Hydroxy, $C_1$-$C_{18}$-Alkyl und/oder $C_1$-$C_{18}$-Alkoxy substituiertes Phenyl;

$R_5$ $C_1$-$C_{18}$-Alkyl; $C_1$-$C_{18}$-Alkoxy; Halogen; Hydroxy; einen Rest der Formel

(3a) $-(O-CH_2-CH_2-)_t-R_6$;

oder einen Rest der Formel

(3b)

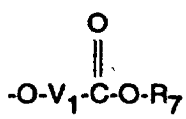

$R_7$ $C_1$-$C_5$-Alkyl oder $C_1$-$C_5$-Alkoxy-$C_1$-$C_5$-Alkyl;

$V_1$ einen $C_1$-$C_4$-Alkylenrest;

$m_1$ 0, 1 oder 2;

t 1 bis 5; und

$R_8$ Wasserstoff; oder $C_1$-$C_5$-Alkyl bedeuten.

Stellen die Substituenten $R_1$ bis $R_6$ eine Alkylgruppe dar, so kann sie geradkettig oder verzweigt sein. Beispiele solcher Alkylreste sind Methyl, Ethyl, n-Propyl, Isopropyl, n-Butyl, Isobutyl, sek.Butyl, tert.Butyl, Pentyl, Isopentyl, tert. Pentyl, n-Hexyl, 2-Ethylhexyl, n-Heptyl, n-Octyl, Isooctyl, n-Nonyl, Isononyl, n-Dodecyl, Heptadecyl oder Octadecyl.

Beispiele für $C_1$-$C_{18}$-Alkoxy oder $C_1$-$C_5$-Alkylthio sind z. B. Methoxy, Ethoxy, Propoxy, Isopropoxy, Butoxy, Isobutoxy, sek.-Butoxy, tert.-Butoxy, Pentoxy, Isopentoxy, n-Heptyloxy, n-Octyloxy, Isooctyloxy, n-Nonyloxy, Isononyloxy, Decyloxy, n-Dodecyloxy, Heptadecyloxy oder Octadecyloxy, bzw. Methylthio, Ethylthio, Propylthio, Isopropylthio, Butylthio, Isobutylthio, sek.-Butylthio, tert.-Butylthio, Pentylthio, Isopentylthio oder tert. Pentylthio.

Beispiele für Monoalkylamino sind Monomethyl-, Monoethyl-, Monopropyl-, Monoisopropyl-, Monobutyl- oder Monopentylamino. Als Beispiele für Dialkylamino seien Dimethyl-, Methylethyl- oder Diethylamino erwähnt.

Halogen bedeutet beispielsweise Fluor, Brom oder vorzugsweise Chlor.

Wichtige s-Triazin-Verbindungen entsprechen der Formel

(4)

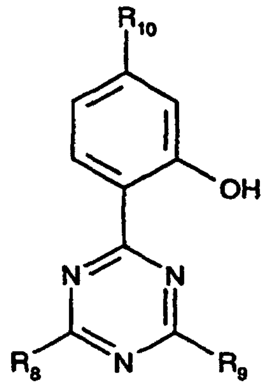

worin

$R_8$ und $R_9$, unabhängig voneinander, nicht substituiertes oder durch $C_1$-$C_5$-Alkyl und/oder $C_1$-$C_5$-Alkoxy substituiertes Phenyl; und
$R_{10}$ Wasserstoff oder $C_1$-$C_5$-Alkyl;

bedeuten.

Weitere interessante Triazinverbindungen entsprechen der Formel

(5)

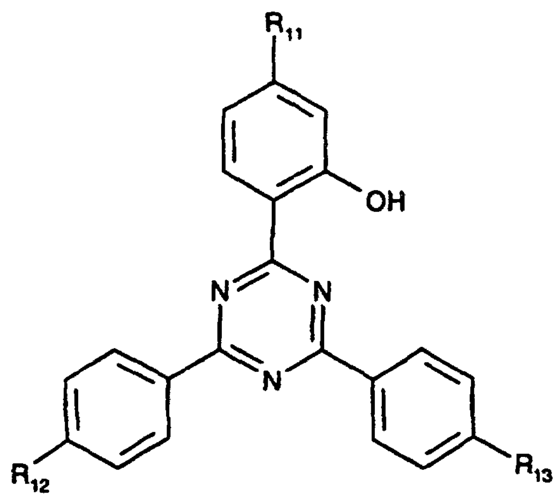

worin

$R_{11}$ Wasserstoff, Hydroxy, $C_1$-$C_{15}$-Alkyl, $C_1$-$C_{15}$-Alkoxy oder einen Rest der Formel (3b), und
$R_{12}$ und $R_{13}$, unabhängig voneinander, Wasserstoff oder $C_1$-$C_{15}$-Alkoxy bedeuten.

Weiterhin sind Hydroxyphenyl-s-triazine der Formel (5) bevorzugt, worin

$R_{11}$, $R_{12}$ und $R_{13}$, unabhängig voneinander, $C_5$-$C_{15}$-Alkoxy, oder solche Verbindungen, bei denen
$R_{11}$ einen Rest der Formel (3b) und
$R_{12}$ und $R_{13}$ $C_5$-$C_{15}$-Alkoxy bedeuten.

Geeignete Verbindungen der Formeln (3), (4) und (5) sind z.B.:

2-(2'-Hydroxy-5'-methylphenyl)-4,6-dimethyl-s-triazin; Fp.= 131°C;
2-(2'-Hydroxy-3',5'-dimethylphenyl)-4,6-dimethyl-s-triazin; Fp.= 177°C;
2-(2'-Hydroxy-4',5'-dimethylphenyl)-4,6-dimethyl-s-triazin; λ=349 µm; T=48 %;

2-(2'-Hydroxy-4',5'-dimethylphenyl)-4,6-diethyl-s-triazin; Fp.= 98°C;
2-(2'-Hydroxy-5'-chlorphenyl)-4,6-dimethyl-s-triazin; Fp.= 160°C;
2-(2'-Hydroxyphenyl)-4,6-dimethyl-s-triazin; Fp.= 133°C;
2-(2'-Hydroxy-5'-tert.-butylphenyl)-4,6-dimethyl-s-triazin; λ=352 µm; T=60 %;
2-(2'-Hydroxyphenyl)-4,6-didecyl-s-triazin; Fp.= 53°C;
2-(2'-Hydroxyphenyl)-4,6-dinonyl-s-triazin; Fp.= 45°C;
2-(2'-Hydroxyphenyl)-4,6-diheptadecyl-s-triazin; λ=338 µm; T=80 %;
2-(2'-Hydroxyphenyl)-4,6-dipropyl-s-triazin; Fp.= 18 bis 20°C;
2-(2'-Hydroxyphenyl)-4,6-bis-(β-methylmercaptoethyl)-s-triazin; λ=341 µm; T=60 %;
2-(2'-Hydroxyphenyl)-4,6-bis-(β-dimethylaminoethyl)-s-triazin; λ=340 µm; T 63 %;
2-(2'-Hydroxyphenyl)-4,6-bis-(β-butylaminoethyl)-s-triazin; λ=341 µm; T=66 %;
2-(2'-Hydroxyphenyl)-4,6-di-tert.-butyl-s-triazin; λ=338 µm; T=68 %;
2-(2'-Hydroxyphenyl)-4,6-dioctyl-s-triazin; Fp.= 40°C;
2-(2'-Hydroxy-4'-methoxyphenyl)-4,6-diphenyl-s-triazin; Fp.= 204-205°C;
2-(2'-Hydroxy-4'-ethoxyphenyl)-4,6-diphenyl-s-triazin; Fp.= 201-202°C;
2-(2'-Hydroxy-4'-isopropyl)-4,6-diphenyl-s-triazin; Fp.= 181-182°C;
2-(2'-Hydroxyphenyl)-4,6-bis(4-methoxyphenyl)-1,3,5-triazin;
2-(2',4'-Dihydroxyphenyl)-4,6-bis(4-methoxyphenyl)-1,3,5-triazin;
2-(2'-hydroxy-3'-methylphenyl)-4,6-bis(4-methoxyphenyl)-1,3,5-triazin;
2-(2',3'-Dihydroxyphenyl)-4,6-bis(4-methoxyphenyl)-1,3,5-triazin;
2-(2'-Hydroxy-5'-chlorophenyl)-4,6-bis(4-methoxyphenyl)-1,3,5-triazin;
2-(2'-Hydroxy-4-methoxyphenyl)-4,6-bis(4-methoxyphenyl)-1,3,5-triazin;
2-(2',4'-Dihydroxyphenyl)-4,6-bis(4-methoxyphenyl)-1,3,5-triazin;
2-(2'-Hydroxy-4-Hexyloxyphenyl)-4,6-bis(4-methoxyphenyl)-1,3,5-triazin;
2-(2'-Hydroxy-4-hexyloxyphenyl)-4,6-bis(3-methoxyphenyl)-1,3,5-triazin;
2-(2-Hydroxy-4-methoxyphenyl)-4-(4-methoxyphenyl)-6-phenyl-1,3,5-triazin;
2-(2'-Hydroxy-4'-[2-ethylhexyloxy])-4,6-bis-(2-ethylhexyloxy)-phenyl-1,3,5-triazin;
2-(2'-Hydroxyphenyl)-4,6-bis(4-methoxyphenyl)-1,3,5-triazin;
2-(2',4'-Dihydroxyphenyl)-4,6-bis(4-methoxyphenyl)-1,3,5-triazin;
2-(2'-hydroxy-3'-methylphenyl)-4,6-bis(4-methoxyphenyl)-1,3,5-triazin;
2-(2',3'-Dihydroxyphenyl)-4,6-bis(4-methoxyphenyl)-1,3,5-triazin;
2-(2'-Hydroxy-5'-chlorophenyl)-4,6-bis(4-methoxyphenyl)-1,3,5-triazin;
2-(2'-Hydroxy-4-methoxyphenyl)-4,6-bis(4-methoxyphenyl)-1,3,5-triazin;
2-(2',4'-Dihydroxyphenyl)-4,6-bis(4-methoxyphenyl)-1,3,5-triazin;
2-(2'-Hydroxy-4-Hexyloxyphenyl)-4,6-bis(4-methoxyphenyl)-1,3,5-triazin;
2-(2'-Hydroxy-4-hexyloxyphenyl)-4,6-bis(3-methoxyphenyl)-1,3,5-triazin;
2-(2-Hydroxy-4-methoxyphenyl)-4-(4-methoxyphenyl)-6-phenyl-1,3,5-triazin;
2-(2'-Hydroxy-4'-[2-ethylhexyloxy])-4,6-bis-(2-ethylhexyloxy)-phenyl-1,3,5-triazin;
4,6-Bis(2-hydroxy-4-methoxyphenyl)-2-(2-propoxy-ethoxy)-1,3,5-triazin;
4,6-Bis(2-hydroxy-4-propoxyphenyl)-2-(2-methoxy-ethoxy)-1,3,5-triazin;
4,6-Bis(2-hydroxy-4-ethoxyphenyl)-2-(2-methoxy-ethoxy)-1,3,5-triazin;
4,6-Bis(2-hydroxy-4-methoxyphenyl)-2-(2-methoxy-ethoxy)-1,3,5 -triazin;
4,6-Bis(2-hydroxy-4-methoxyphenyl)-2-(2-ethoxy-ethoxy)-1,3,5-triazin;
4,6-Bis(2-hydroxy-4-methoxyphenyl)-2-[2-(2-ethoxy-ethoxy)-ethoxy]-1,3,5-triazin;
4,6-Bis(2-hydroxy-4-ethoxyphenyl)-2-(2-ethoxy-ethoxy)-1,3,5-triazin;
4,6-Bis(2-hydroxy-4-ethoxyphenyl)-2-[2-(2-ethoxy-ethoxy)-ethoxy]-1,3,5-triazin;
4,6-Bis(2-hydroxy-4-ethoxyphenyl)-2-(2-ethoxy-2-methylethoxy)-1,3,5-triazin;
4,6-Bis(2-hydroxy-4-ethoxyphenyl)-2-(ethoxy-methoxy)-1,3,5-triazin;
4,6-Bis(2-hydroxy-4-methoxyphenyl)-2-octyloxy-1,3,5-triazin;
4,6-Bis(2-hydroxy-4-methoxyphenyl)-2-{2-[2-(2-ethoxy)ethoxy]ethoxy}ethoxy-triazin;
4,6-Bis(2-hydroxy-4-ethoxyphenyl)-2-{2-[2-(2-ethoxy)ethoxy]ethoxy}ethoxy-1,3,5-triazin; und
4,6-Bis(2-hydroxy-4-ethoxyphenyl)-2-butoxy-1,3,5-triazin.

(T = prozentuale Transmission einer Lösung von 1 mg Substanz in 100 ml Chloroform bei 1 cm Schichtdicke; λ [µm] ist der maximale Extinktionskoeffizient).

Die Verbindungen der Formeln (3), (4) und (5) sind bekannt und können in an sich bekannter Weise hergestellt werden, so z.B. durch Erhitzen eines Amidins und eines o-Hydroxybenzolcarbonsäureesters, vorzugsweise im ungefähren molaren Mengenverhältnis von 2:1 in siedenden, organischen Lösungsmitteln [cf. US 3,896,125 und Helv. Chim.

Acta 55, 1566-1595 (1972)].

Weiterhin können als ($Q_2$) Strukturelemente von Triazin-UV-Absorbern der Formel

(6)

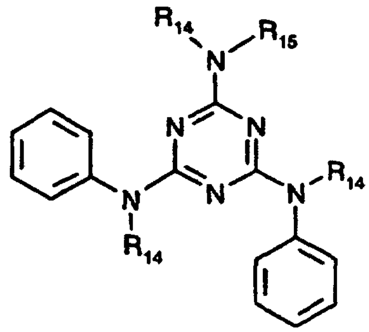

verwendet werden.

$R_{14}$ und $R_{15}$ bedeuten dabei, unabhängig voneinander, Wasserstoff, Hydroxy, $C_1$-$C_5$-Alkyl oder $C_1$-$C_5$-Alkoxy.

Bei den Benzotriazolen $Q_3$ handelt es sich um Verbindungen der Formel

(7)

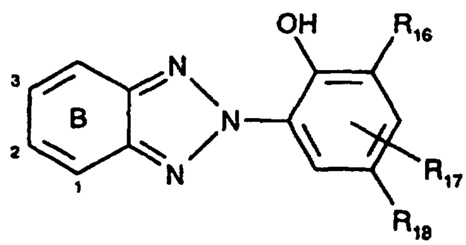

worin

$R_{16}$ und $R_{18}$, unabhängig voneinander, Wasserstoff; nicht substituiertes oder durch Phenyl substituiertes $C_1$-$C_{18}$-Alkyl oder $C_1$-$C_8$-Alkoxy; Halogen oder die Gruppe

(7a)

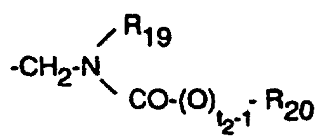

bedeuten, worin

$R_{19}$ Wasserstoff; $C_1$-$C_{10}$-Alkyl; $C_5$-$C_8$-Cycloalkyl; $C_7$-$C_{10}$-Aralkyl; oder $C_8$-$C_{10}$Aryl;
$R_{20}$ Wasserstoff; $C_1$-$C_{20}$-Alkyl; $C_2$-$C_{17}$-Alkenyl; $C_5$-$C_8$-Cycloalkyl; $C_7$-$C_{10}$-Aralkyl; $C_6$-$C_{10}$Aryl; und
$t_2$ 1 oder 2 bedeuten, und, sofern $t_2$= 1 ist,

$R_{19}$ und $R_{20}$ zusammen mit dem Ringbrückenglied

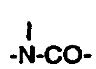

auch einen ein- oder mehrkernigen stickstoffhaltigen Heterocyclus bilden können und in diesem Falle $R_{19}$-COoder unsubstituiertes oder durch $C_1$-$C_5$Alkyl substituiertes Methylen und

$R_{20}$ $C_2$-$C_5$Alkylen, $C_2$-$C_5$Alkenylen, $C_6$-$C_{10}$Arylen oder vicinal gebundenes Di-, Tetra- oder Hexahydro-$C_6$-$C_{10}$Arylen bedeuten;

$R_{17}$ $C_1$-$C_{18}$-Alkyl; $C_1$-$C_{18}$-Alkoxy; Halogen; $C_6$-$C_{10}$Aryl; $C_7$-$C_{10}$Aralkyl; oder $C_5$-$C_8$Cycloalkyl; und

der Ring B in den Stellungen 1, 2 und 3 durch $C_1$-$C_5$-Alkyl, $C_1$-$C_5$-Alkoxy, Carboxy, $C_2$-$C_9$-Alkoxycarbonyl, $H_2NCO$-, $SO_2$-, $C_1$-$C_5$-Alkylsulfonyl, Halogen oder durch den Rest der Formel

(7b) 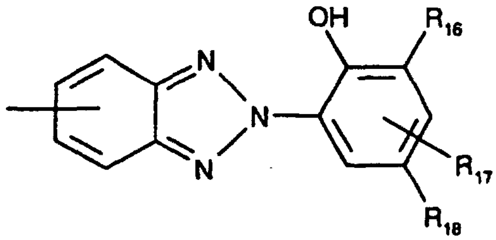

substituiert sein kann.

Die Substituenten $R_{19}$ und $R_{20}$ in der Bedeutung von $C_1$-$C_{10}$-Alkyl bzw. $C_1$-$C_{20}$-Alkyl können geradkettige oder verzweigte Kohlenwasserstoffreste sein, wie z.B. Methyl, Ethyl, n-Propyl, Isopropyl, n-Butyl, Isobutyl, sek.Butyl, tert.Butyl, Pentyl, Isopentyl, tert.Pentyl, n-Hexyl, 2-Ethylhexyl, n-Heptyl, n-Octyl, Isooctyl, n-Nonyl, Isononyl, n-Decyl, n-Dodecyl, Heptadecyl, Octadecyl oder Eicosyl.

Bedeuten $R_{19}$ und $R_{20}$ $C_6$-$C_{10}$-Aryl, dann kann es ein mono- oder bicyclischer aromatischer Rest, wie z.B. Phenyl oder Naphthyl sein.

Als $C_7$-$C_{10}$-Aralkyl bedeuten $R_{19}$ und $R_{20}$ z.B. Benzyl, Phenethyl, $\alpha$-Methylphenethyl oder $\alpha,\alpha$-Dimethylbenzyl.

Stellen $R_{19}$ und $R_{20}$ $C_5$-$C_8$-Cycloalkyl dar, so kann es Cyclopentyl, Cycloheptyl, Cyclooctyl oder vorzugsweise Cyclohexyl sein.

$R_{20}$ als $C_2$-$C_{17}$-Alkenylreste sind z.B. Vinyl, Allyl, 1-Propenyl, 2-Butenyl, 2-Pentenyl, 2-Hexenyl, 2-Decenyl, 3,6,8-Decatrienyl oder 2-Heptadecenyl.

Bevorzugte Benzotriazolverbindungen entsprechen der Formel

(8) 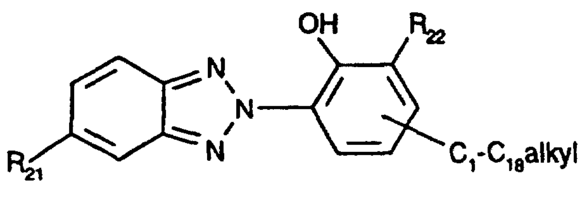

worin

$R_{21}$ $C_1$-$C_{18}$-Alkyl, oder vorzugsweise Wasserstoff; und
$R_{22}$ nicht substituiertes oder durch Phenyl substituiertes $C_1$-$C_{18}$-Alkyl bedeuten.

Beispielhafte Benzotriazolverbindungen der Formeln (7) und (8) sind:

2-(2'-Hydroxy-5'-methylphenyl)-benzotriazol;
2-(2'-Hydroxy-5-tert.butylphenyl)-benzotriazol;
2-(2'-Hydroxy-3'-tert.butyl-5'-methylphenyl)-5-chlorbenzotriazol;
2-(2'-Hydroxy-3'-5'-di-tert.butylphenyl)-5-chlor-benzotriazol;
2-(2'-Hydroxy-5'-tert.octylphenyl)-benzotriazol;
2-(2'-Hydroxy-3'-acryloylamidomethyl-5'-methylphenyl)-benzotriazol;
2-(2'-Hydroxy-3'-acryloylamidomethyl-5'-benzylphenyl)-benzotriazol;
2-(2'-Hydroxy-3'-butoxacetamidomethyl-5'-benzylphenyl)-benzotriazol;
2-(2'-Hydroxy-3'5'-di-tert.amylphenyl)-benzotriazol; und
2-(2'-Hydroxy-3',5'-di-tert.butylphenyl)-benzotriazol.

Die als UV-absorbierend bekannten Benzotriazolverbindungen der Formeln (7) und (8) sind grösstenteils in

der FR-A-1,195,307 oder der US-A-3,629,192 beschrieben.

Bei den Benzophenonen ($Q_4$) handelt es sich um Verbindungen der Formel

(9)

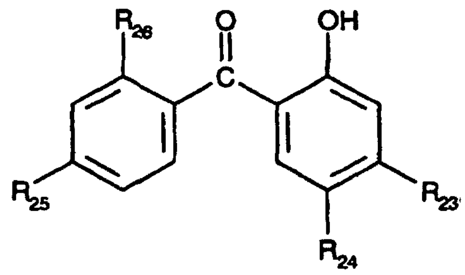

worin

$R_{23}$ Wasserstoff, Hydroxy, $C_1$-$C_{14}$-Alkoxy, Phenoxy oder Amino; wobei $C_1$-$C_{14}$-Alkoxy durch einen Rest der Formel

(9a)

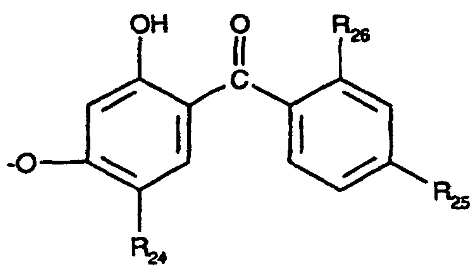

und/oder einen Acyloxyrest substituiert sein kann;

$R_{24}$ Wasserstoff, Halogen oder $C_1$-$C_5$Alkyl;

$R_{25}$ Wasserstoff, Hydroxy oder $C_1$-$C_5$-Alkoxy; und

$R_{26}$ Wasserstoff oder Hydroxy;

bedeuten.

Acyl bedeutet $C_1$-$C_5$-Alkanoyl, wie z.B. Formyl, Acetyl, Propionyl, Acryloyl, Methacryloyl oder Benzoyl.

Die Verbindungen der Formel (9) können nach an sich bekannten Verfahren, wie sie z.B. in der US-A-3,468,938, US-A-3,696,077 bzw. US-A-4,698,064 beschrieben sind, hergestellt werden.

Bei den UV-Chromophoren ($Q_5$) handelt es sich um Paraaminobenzoesäurederivate der Formel

(10)

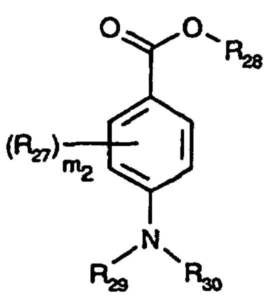

,

worin

$R_{27}$ Hydroxy; Halogen; Cyano; $C_1$-$C_5$-Alkyl; $C_1$-$C_5$-Alkoxy; Mono-$C_1$-$C_5$-Alkylamino; Di-$C_1$-$C_5$-Alkylamino;

$R_{28}$ Wasserstoff; $C_1$-$C_5$-Alkyl;

$R_{29}$ und $R_{30}$, unabhängig voneinander, Wasserstoff; oder $C_1$-$C_5$-Alkyl; und

$m_2$ 0, 1 oder 2;

bedeuten.

Vorzugsweise werden als UV-Chromophore ($Q_5$) Strukturelemente eingesetzt, sie sich von der Paraaminobenzoesäure oder deren Methyl- oder Ethylester ableiten.

Als UV-Chromophore ($Q_6$) kommen Benzylidencampher in Betracht. Sie entsprechen der Formel

(11) 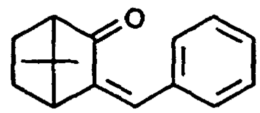 .

Für die Herstellung der erfindungsgemässen liposomogenen UV-Absorber werden die in Betracht kommenden UV-Chromophore ($Q_1$)-($Q_6$), sofern es sich dabei um in Wasser schwerlösliche Verbindungen handelt, als wässrige Dispersionen eingesetzt.

Als geeignete Dispergatoren kommen dabei verschiedene Verbindungen in Betracht, wie z.B. saure Ester oder deren Salze von Alkylenoxidaddukten, Polystyrolsulfonate, Fettsäuretauride, alkylierte Diphenyloxid-mono- oder -di-sulfonate, Sulfonate von Polycarbonsäureestern, oder die mit einer organischen Dicarbonsäure, oder einer anorganischen mehrbasischen Säure in einen sauren Ester übergeführten Anlagerungsprodukte von 1 bis 60, vorzugsweise 2 bis 30 Mol, Ethylenoxid und/oder Propylenoxid an Fettamine, Fettamide, Fettsäuren oder Fettalkohole mit je 8 bis 22 Kohlenstoffatomen oder an drei- bis sechswertige Alkanole mit 3 bis 6 Kohlenstoffatomen, Ligninsulfonate und Formaldehyd-Kondensationsprodukte. Einzelheiten über die Dispergatoren sowie die Herstellung der UV-Absorberdispersionen sind z.B. in der EP-A-0,523,006 zu finden.

Bevorzugt werden erfindungsgemäss folgende Chromophore Q eingesetzt:

(2a) 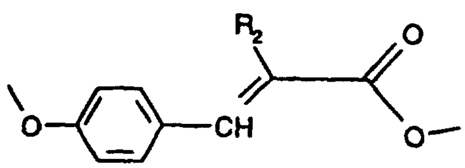 ; (6a) 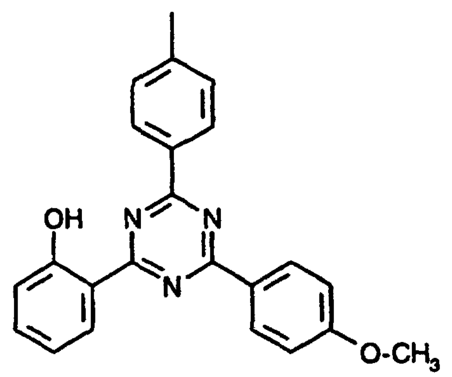

bzw.

(7a) 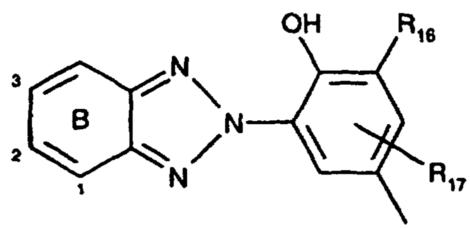 ;

$R_2$, $R_{14}$, $R_{16}$, $R_{17}$ und B haben dabei die in den Formeln (2), (6) bzw.(7) angegebenen Bedeutungen.

Bei dem organischen Rest W handelt es sich in der Regel um einen mindestens zweiwertigen Alkylenrest, der gegebenenfalls durch eine Carbonyl-, Carbonylato- oder Ethergruppe unterbrochen sein kann. W stellt insbesondere eine verzweigte oder vorzugsweise geradkettige, 1 bis 8, vorzugsweise 2 bis 5 Kohlenstoffatome aufweisende Alkylengruppe dar. Es handelt sich beispielsweise um die $-CH_2-$;$-CH_2CH_2-$;

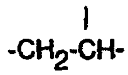 , 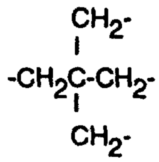 ,

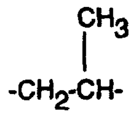 ;

$-CH_2CH_2CH_2-$; $-CH_2CH_2CH_2CH_2-$;

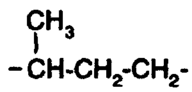 ; 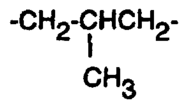 ;

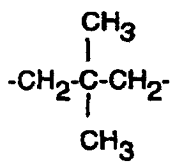 ;

$-CH_2(CH_2)_4CH_2-$; $-CH_2(CH_2)_6CH_2-$; $-CH_2-(CO)O-CH_2$; $-CH_2-(CO)-CH_2$; $-(CO)O-CH_2-$ oder die $-CH=CH-CH_2-$; -Gruppe.

Als hydrophile Reste Z1 und Z2 kommen vorzugsweise

($Z_a$) Ammonium- oder Aminverbindungen;
($Z_b$) Phosphatverbindungen;
($Z_c$) Carboxylatverbindungen;
($Z_d$) Polyole; und
($Z_e$) Sulfatverbindungen

in Betracht.

Sie bilden die hydrophilen Kopfgruppen der erfindungsgemässen liposomogenen UV-Absorber. Bei diesen Strukturelementen kann man zwischen kationischen ($Z_a$), anionischen (($Z_b$), ($Z_c$), ($Z_e$)) und neutralen Kopfgruppen (($Z_a$) und ($Z_d$)) unterscheiden.

Als kationische Ammoniumverbindungen ($Z_a$) kommen insbesondere Mono- oder Di-$C_1$-$C_5$-Alkylammoniumverbindungen, die durch weitere Substituenten modifiziert werden können, in Betracht. Beispielhaft seien die folgenden Gruppen erwähnt:

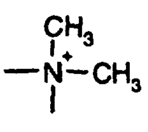 ; 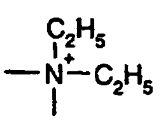 ; 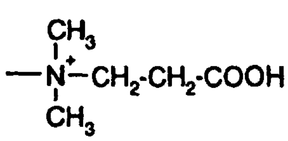 ; 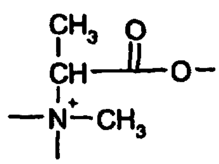 ;

oder

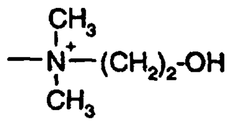

Als neutrale Aminverbindungen kommen z.B. die Gruppen

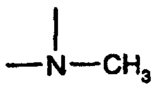 ; 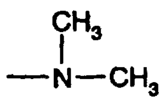 ; 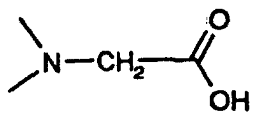 oder 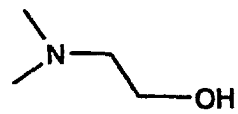

in Betracht.

Als Phosphatgruppen $Z_b$ kommen insbesondere Verbindungen in Betracht, die sich von Mono- und Diestern der Phosphorsäure ableiten. Die Phosphorverbindungen werden dabei gewöhnlich in Form ihrer Natriumsalze verwendet.

Die Carboxylatverbindungen ($Z_c$) leiten sich von niederen Mono- oder Dicarbonsäuren ab und werden ebenfalls gewöhnlich als Natriumsalze verwendet.

Als Polyole ($Z_d$) kommen z.B. die folgenden Verbindungen in Betracht:

(12a) 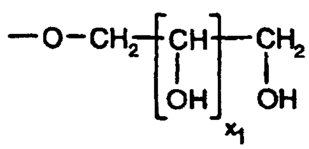 ; (12b) 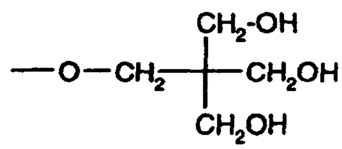 ;

$X_1$ = 1, 2, 3 oder 4

(12c) 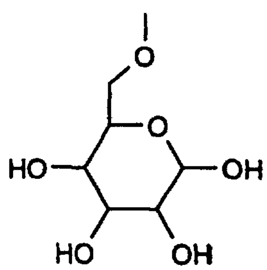 ; (12d) 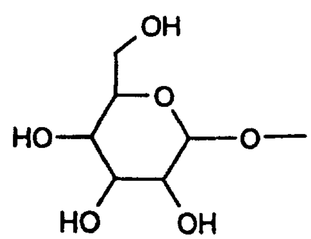 ;

und

(12e) 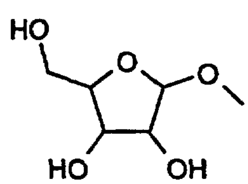 .

Die Sulfatverbindungen ($Z_e$) leiten sich von Alkylsulfaten ab, die gegebenenfalls noch weiter substituiert sein

können. Als Sulfatgruppen kommen in erster Linie das Sulfation, $SO_4^{2-}$, in Betracht.

Je nachdem, welche Ausgangsverbindungen man verwendet, können die Kopfgruppen $(Z_a)$-$(Z_e)$ gleichzeitig mit dem organischen Rest W die Einheit $-W-Z_1$ bzw. $-W-Z_2-$ bilden.

Die erfindungsgemäss eingesetzten liposomogenen UV-Absorber lassen sich nach an sich bekannten Methoden herstellen. In der Regel erfolgt die Herstellung durch eine planmässige Folge von an sich bekannten Reaktionsschritten wie Kondensation, Alkylierung, Veresterung, Verseifung usw.. Angaben zur Durchführung der Reaktionen können aus Monographien zur organischen Synthese, wie z.B. J. March, Advanced Organic Chemistry, 2nd Edition, McGraw-Hill, New York, 1977, entnommen werden.

Die Einführung der hydrophoben Reste A kann auf sehr unterschiedliche Weise erfolgen, wie z.B. durch Veretherung der phenolischen OH-Gruppe des UV-Chromophors (vgl. Beispiele 1, 2, 5 und 7) oder durch Umsetzung eines Säurechlorids mit einem langkettigen Dialkylamin (vgl. Beispiel 4). Zur Einführung der hydrophilen Kopfgruppen Z eignen sich ebenfalls eine Vielzahl von Reaktionen, wie aus den Beispielen 1 und 2 (Quaternierung) ersichtlich ist. Bei geeigneter Reaktionsführung können A und Z aber auch gleichzeitig, d.h. in einem Reaktionsschritt, eingeführt werden. Dies gelingt z.B. durch Öffnung eines geeigneten Säureanhydrids mit einem Fettalkohol (Beispiel 6).

Ganz besonders bevorzugt sind liposomogene Verbindungen der Formel

$$(13) \qquad [(A_1)_{n_1}(Q)_p(W)_q]_{s_2}\text{-}(Z_1)_{r_1}$$

worin

- $A_1$ einen hydrophoben Rest;
- Q einen UV-Chromophor;
- W einen organischen Rest;
- $Z_1$ einen hydrophilen Rest;
- $n_1$ eine Zahl von 1 bis 4;
- p 1 oder 2;
- q 1 bis 3;
- $r_1$ 1 oder 2; und
- $s_2$ 1 bis 3;

bedeuten.

Weiterhin sind Verbindungen der Formel

(14) 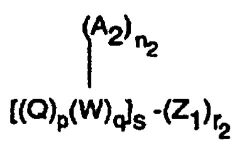

bevorzugt, worin

- $A_2$ einen hydrophoben Rest;
- Q einen UV-Chromophor;
- W einen organischen Rest;
- $Z_1$ einen hydrophilen Rest;
- $n_2$ 1 oder 2;
- p 1 oder 2;
- q 1 bis 3;
- $r_2$ 1 oder 2; und
- s 1 oder 2;

bedeuten.

Von den erfindungsgemässen liposomogenen UV-Absorber, die als UV-Chromophor ein Strukturelement eines Zimtsäurederivates enthalten, sind die Verbindungen der Formel

(15) 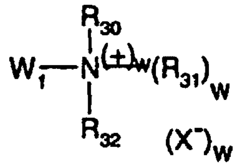

bevorzugt, worin

| | |
|---|---|
| $W_1$ | einen Rest der Formel |

(15a) 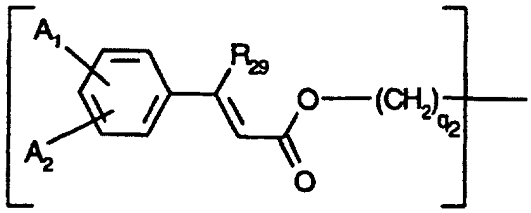

| | |
|---|---|
| $R_{29}$ | Wasserstoff; $C_1$-$C_4$-Alkyl oder -CN; |
| $R_{30}$ und $R_{31}$ | unabhängig voneinander, $C_1$-$C_5$-Alkyl, Hydroxy, Hydroxy-$C_1$-$C_5$-Alkyl, Carboxy; |
| $R_{32}$ | $C_1$-$C_5$-Alkyl; Hydroxy; Hydroxy-$C_1$-$C_5$-Alkyl; Carboxy; oder einen Rest der Formel (15a); |
| $A_1$ und $A_2$, | unabhängig voneinander, Wasserstoff oder einen $C_{10}$-$C_{14}$-Alkoxyrest, wobei ein Rest immer einen $C_{10}$-$C_{14}$-Alkoxyrest bedeutet; |
| X | ein Halogenatom; |
| q | eine Zahl von 2 bis 4; und |
| w | 0 oder 1; |

bedeuten, oder Verbindungen der Formel

(16) 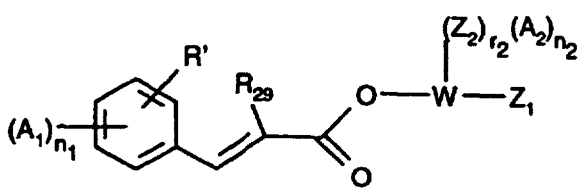

worin

| | |
|---|---|
| $R_{29}$ | Wasserstoff; $C_1$-$C_4$-Alkyl oder -CN; |
| R' | Wasserstoff, $C_1$-$C_4$-Alkyl; oder $C_1$-$C_4$-Alkoxy; |
| $A_1$ und $A_2$, | unabhängig voneinander, einen $C_{10}$-$C_{14}$-Alkoxyrest; |
| W | einen gegebenenfalls durch eine -O(CO)-Gruppe unterbrochenen $C_2$-$C_4$-Alkylenrest; und |
| $Z_1$ und $Z_2$, | unabhängig voneinander, den Rest einer $C_1$-$C_3$-Carbonsäure; |
| $n_1$ und $n_2$, | unabhängig voneinander, 0, 1 oder 2, wobei $n_1$=$n_2$=0 nicht mitumfasst ist; und |
| $r_2$ | 1 oder 2 bedeuten. |

Bevorzugte erfindungsgemässe liposomogene UV-Absorber, die als UV-Chromophor einen Triazinrest aufweisen, entsprechen der Formel

(17) 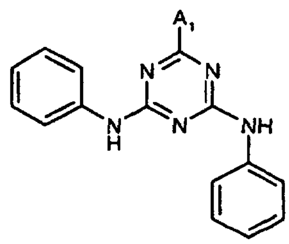

worin

$A_1$ einen Rest der Formel

(17a) 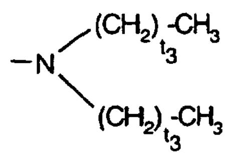

oder der Formel

(17b) $O-(CH_2)_{t_4}-CH_3$

und

$t_3$ und $t_4$, unabhängig voneinander, eine Zahl von 5 bis 13;

bedeuten.

Bevorzugte erfindungsgemässe liposomogene UV-Absorber, die als UV-Chromophor einen Benzylidencampher aufweisen, entsprechen der Formel

(18a) 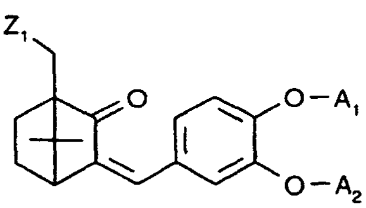

oder der Formel

(18b) 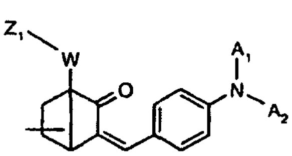

worin

$A_1$, $A_2$, W und $Z_1$ die in Formel (1) angegebene Bedeutung haben.

Die erfindungsgemäss eingesetzten liposomogenen UV-Absorber zeichnen sich dadurch aus, dass sie

- eine hohe Substantivität zu Human-Haar und haben und
- einen hohen UV-Schutz für das Haar garantieren.

Einen weiteren Erfindungsgegenstand bildet ein haarkosmetisches Präparat, enthaltend mindestens 0,25 bis 5 Gew.%, bezogen auf das Gesamtgewicht der Zusammensetzung, eines liposomogenen UV-Absorbers der Formel (1) sowie mindestens einen haut- und haarverträglichen Hilfsstoff.

Eine haarkosmetische Zusammensetzung, enthaltend mindestens 0,25 bis 5 Gew.%, bezogen auf das Gesamtgewicht der Zusammensetzung, eines liposomogenen UV-Absorbers der Formel (1), kann durch physikalisches Mischen des oder der liposomogenen UV-Absorber mit dem Hilfsstoff oder den Hilfsstoffen durch gewöhnliche Methoden, wie z.B. durch einfaches Zusammenrühren der Einzelkomponenten hergestellt werden.

Für die haarkosmetische Verwendung haben die erfindungsgemässen UV-Absorber, sofern sie nicht wasserlöslich sind, gewöhnlich eine mittlere Partikelgrösse im Bereich von 0,02 bis 2, vorzugsweise 0,05 bis 1,5, und ganz besonders von 0,1 bis 1,0 µm. Die unlöslichen erfindungsgemässen UV-Absorber können durch übliche Methoden, z. B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe, wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Für die Herstellung der erfindungsgemässen haarkosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Das haarkosmetische Präparat kann dabei

- in Form eines Schampoo, einer Lotion, eines Gels oder einer Emulsion zur Spülung, vor oder nach dem Shampoonieren, vor oder nach einer Färbung oder Entfärbung, vor oder nach einer Dauerwelle oder einem Entfrisiervorgang,
- in Form einer Lotion, eines Schaums oder eines Gels zum Frisieren oder Behandeln,
- in Form einer Lotion oder eines Gels zum Bürsten oder zu Wellengebung,
- in Form eines Haarlacks,
- in Form einer Zusammensetzung zur Dauerwelle oder zum Entfrisieren, zur Färbung oder Entfärbung der Haare vorliegen.

Es können zum Beispiel die folgenden haarkosmetischen Formulierungen verwendet werden:

$a_1$) spontan emulgierende Stammformulierung, bestehend aus dem UV-Absorber, PEG-6-$C_{10}$-Oxoalkohol und Sorbitansesquioleat, das mit Wasser und einer beliebigen quaternären Ammoniumverbindung, wie z.B. 4% Minkamidopropyl-dimethyl-2-hydroxyethylammoniumchlorid oder Quaternium 80 versetzt wird;

$a_2$) spontan emulgierende Stammformulierung bestehend aus dem UV-Absorber, Tributylcitrat und PEG-20-Sorbitanmonooleat, das mit Wasser und einer beliebigen quaternären Ammoniumverbindung, wie z.B. 4% Minkamidopropyl-dimethyl-2-hydroxyethylammoniumchlorid oder Quaternium 80 versetzt wird;

b) Quatdotierte Lösungen des UV-Absorbers in Butyltriglykol und Tributylcitrat;

c) Dispersionen von nach bekannten Methoden (Fällen aus Lösungen oder Lösungsgemischen, Mahlungen) erhaltenen mikronisierten UV-Absorbern mit einem mittleren Durchmesser von 0,05 - 1,0 mm in APG ( z.B. Plantaren) und einem Quat (z.B. Minkamidopropyl-dimethyl-2-hydroxyethylammoniumchlorid) in einer wässrigen Zubereitung)

d) Mischungen oder Lösungen des UV-Absorbers mit N-Alkylpyrrolidon.

Das haarkosmetische Präparat kann neben den erfindungsgemässen liposomogenen UV-Absorbern auch noch einen oder mehrere weitere UV-Schutzstoffe, wie z.B. Oxanilide oder Vinylgruppen enthaltende Amide oder Zimtsäureamide enthalten. Solche Schutzstoffe sind z.B. in der GB-A-2,286,774 beschrieben oder auch aus Cosmetics & Toiletries (107), 50ff (1992) bekannt.

Weiterhin können die erfindungsgemässen Zusammensetzungen noch weitere nützliche Hilfsmittel, wie z.B. oberflächenaktive Mittel, Verdickungsmittel, Polymere, Konservierungsmittel, Duftstoffe, Schaumstabilisatoren, Elektrolyte, organische Lösungsmittel, Öle, Wachse, Entfettungsmittel, Farbstoffe und/oder Pigmente enthalten, die dazu dienen, der haarkosmetischen Zusammensetzung die gleiche Tönung wie das zu behandelnde Haar zu verleihen sowie weitere, in der Haarkosmetik übliche Hilfsstoffe.

Die vorliegende Erfindung betrifft auch ein Verfahren zur Behandlung von Human-Haar zum Schützen vor

der schädigenden Einwirkung von UV-Strahlung. Das Verfahren ist dadurch gekennzeichnet, dass man das Haar mit einem Schampoo, einer Lotion, einem Gel oder einer Emulsion zur Spülung, vor oder nach dem Shampoonieren, vor oder nach einer Färbung oder Entfärbung, vor oder nach einer Dauerwelle oder einem Entfrisiervorgang, mit einer Lotion, einem Schaum oder einem Gel zum Frisieren, mit einer Lotion, einem Schaum oder einem Gel zum Bürsten oder zu Wellengebung, mit einem Haarlack, mit einer Zusammensetzung zur Dauerwelle oder zum Entfrisieren, zur Färbung oder Entfärbung der Haare behandelt, wobei das Schampoo, die Lotion, das Gel, die Emulsion, der Haarlack oder die Zusammensetzung zur Dauerwelle einen liposomogenen UV-Absorber der Formel (1) enthält.

Die folgenden Beispiele dienen der Veranschaulichung der Erfindung, ohne sie darauf zu beschränken.

Herstellungsbeispiele der neuen Verbindungen:

Beispiel 1: Bis-{2-[3-(4-dodecyloxy-phenyl)-acryloyloxy]-ethyl}-dimethyl-ammonium-methylsulfat

(101)

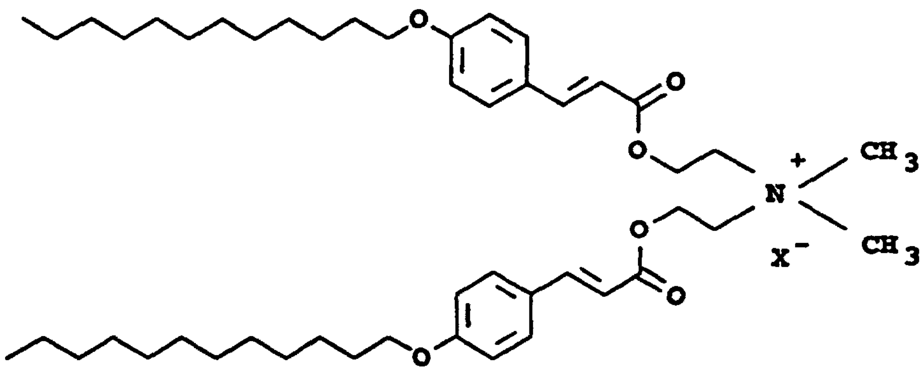

(101a) X=$CH_3SO_4$

(101b) X=I

Die Herstellung von 4-Dodecyloxy-zimtsäure erfolgt nach allgemein bekannten Verfahren durch Kondensation eines 4-Alkoxy-benzaldehyds mit Malonsäure bzw. durch Umsetzung von Cumarsäure mit Dodecylbromid. Die farblosen Kristalle zeigen einen Schmelzpunkt von 158-159°C. Das entsprechende Säurechlorid wird daraus durch Reaktion mit Oxalylchlorid in Benzol (18 Std., Raumtemperatur) nahezu quantitativ erhalten.

28,1g (0.08 Mol) 4-Dodecyloxy-zimtsäurechlorid werden in 150 ml Chloroform vorgelegt und unter Rühren und Kühlung bei Raumtemperatur zunächst mit 4,1 g (0.04 Mol) Triethylamin, dann mit 5,0 g (0.04 Mol) N-Methyldiethanolamin versetzt. Man rührt ca. 4 Stunden nach, kühlt auf 10°C ab und tropft die Reaktionslösung zur Freisetzung der Base in 250 ml Chloroform, welches zuvor mit $NH_3$ gesättigt wurde. Die Suspension wird zweimal über Hyflo® filtriert und zur Trockene eingedampft. Das rohe Amin kann durch Säulenchromatographie gereinigt werden (Kieselgel, Toluol/Ethanol 95/5). Die Ausbeute beträgt 53% d.Th.

Zur Quaternierung wird das Amin in Toluol vorgelegt und bei 40°C mit der molaren Menge Dimethylsulfat (=101a) bzw. Methyliodid (=101b) umgesetzt. Nach 3 Stunden ist das Dünnschichtchromatogramm frei von Edukt. Man dampft im Vakuum zur Trockene ein und versetzt zur Kristallisation mit Aceton. Die farblosen Kristalle werden mit Aceton, dann mit Hexan gewaschen und bei 40°C getrocknet. Die Quaternierung verläuft nahezu quantitativ.

UV-Spektrum ($10^{-5}$M; Ethanol): $\lambda_{max}$ = 313 nm $\varepsilon_{max}$ = 53 500 $M^{-1}cm^{-1}$

Beispiel 2: {2-[3-(3,4-Bis-dodecyloxy-phenyl)-acryloyloxy]-ethyl}-(2-hydroxy-ethyl)-dimethylammonium-bromid

(102) 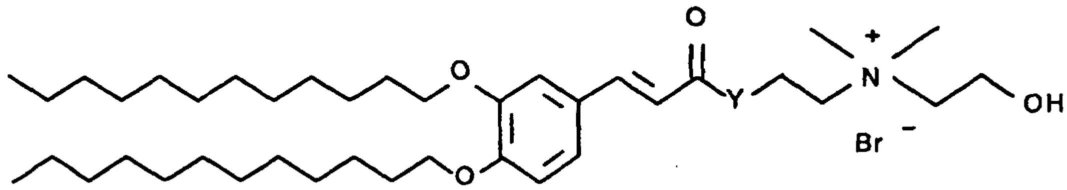

(102a): (Y=O)

(102b): (Y=NH)

Die Herstellung von 3,4-Bis-dodecyloxy-zimtsäure und des entsprechenden Säurechlorids erfolgt in Analogie zu dem in Beispiel 1 beschriebenen Verfahren. Zur Veresterung und Freisetzung der Aminbase wird ebenfalls analog zu Beispiel 1 mit 2-Dimethylaminoethanol umgesetzt.

Die Quaternierung mit 2-Bromethanol wird bei 100°C innerhalb von 12 Stunden in Toluol durchgeführt. Das Rohprodukt kann durch Waschen mit Aceton und nachfolgender Säulenchromatographie weiter gereinigt werden.

UV-Spektrum ($10^{-5}$M; Ethanol): $\lambda_{max}$ = 328 nm $\varepsilon_{max}$ = 19 500 $M^{-1}cm^{-1}$

Beispiel 3: {2-[3-(3,4-Bis-dodecyloxy-phenyl)-acryloylamino]-ethyl}-(2-hydroxy-ethyl)-dimethyl-ammonium-bromid

Die Synthese dieser Verbindung erfolgt in Anlehnung an das in Beispiel 2 beschriebene Verfahren. Anstelle der Veresterung wird mit 2-Dimethylamino-ethylamin zum Amid umgesetzt. Das Produkt lässt sich durch Verreiben mit Aceton und Umkristallisation aus Methanol reinigen.

UV-Spektrum ($10^{-5}$M; Ethanol): $\lambda_{max}$ = 321 nm $\varepsilon_{max}$ = 19 500 $M^{-1}cm^{-1}$

Beispiel 4: 2,4-Bis-[(4-Carboxyl)-anilino]-6-dioctadecylamino-s-triazin (Na-Salz)

(104) 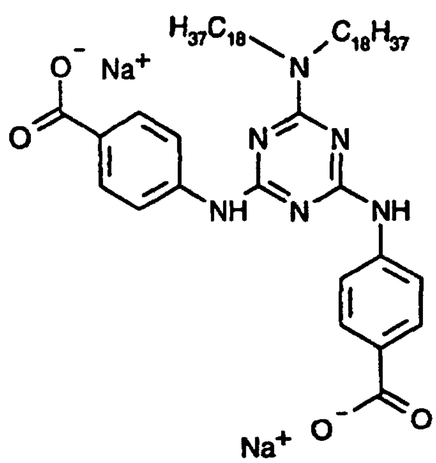

18,5 g (0.1 Mol) Cyanurchlorid werden bei 0-5°C in 250 ml eines Dioxan/Wasser (9/1)-Gemisches vorgelegt. Man trägt unter Rühren 16,5 g (0.1 Mol) 4-Aminobenzoesäure-ethylester ein und lässt die Temperatur auf 15°C ansteigen, wobei der pH-Wert auf 0,5 abfällt. Die weisse Suspension wird mit 30% NaOH auf einen pH-Wert von 8,0-8,5 gestellt und mit einem weiteren Äquivalent Amin versetzt. Man verdünnt mit 250 ml Dioxan/Wasser und erwärmt auf

90°C, wobei der pH-Wert durch automatisches Zudosieren von NaOH bei 8,5 gehalten wird. Nach 4 Stunden wird abgenutscht und mit Dioxan, Wasser und Methanol gewaschen. Das rohe Zwischenprodukt wird durch Umkristallisation aus Ethylcellosolve gereinigt (Ausbeute: 25,7 g; 58% d.Th.; Schmelzpunkt 264°C).

6,63 g (0.015 Mol) des Zwischenprodukts sowie 1,83 g (0.015 Mol) 4-(Dimethylamino)-pyridin werden in 70 ml DMF vorgelegt und bei 60°C mit 8,3 g Dioctadecylamin, gelöst in 30 ml Chloroform, versetzt. Man rührt 3 Stunden bei 100°C, filtriert die heisse Lösung und dampft zur Trockene ein. Der Rückstand wird mit 50 ml Toluol/Essigester (80/20) extrahiert und durch Säulenchromatographie (Kieselgel) gereinigt (Ausbeute: 4,5 g; 33% d.Th.; farbloser, wachsartiger Ester).

Zur Verseifung wird der Ethylester in einem Ethanol/NaOH-Gemisch vorgelegt und 2 Stunden am Rückfluss erhitzt. Der gelartige Niederschlag wird abfiltriert und mit Aceton gewaschen. Die Verseifung erfolgt nahezu quantitativ.

UV-Spektrum ($10^{-5}$M; Ethanol): $\lambda_{max}$ = 301 nm $\varepsilon_{max}$ = 60 000 $M^{-1}cm^{-1}$

Beispiel 5: 2,3-Diacetoxy-bernsteinsäure-mono-{3-[3-(3,4-bis-dodecyloxy-phenyl)acryloyloxy]-2-hydroxy-propyl}-ester

(105)

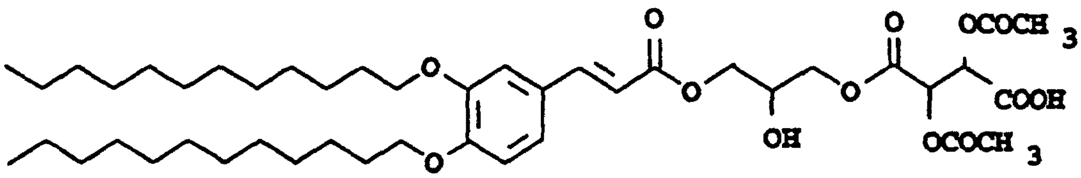

Die Herstellung von 3,4-Bis-dodecyloxy-zimtsäure und des entsprechenden Säurechlorids erfolgt analog dem in Beispiel 1 beschriebenen Verfahren. Das Monoglycerid wird daraus in Anlehnung an eine Vorschrift von Watanabe et al. (J.Med.Chem. 1980,23, 50-59) durch Veresterung mit Isopropylidenglycerin und nachfolgender Abspaltung der Schutzgruppe mit Borsäure in nahezu quantitativer Ausbeute erhalten.

Eine Mischung von 5,91 g (0.01 Mol) Monoglycerid und 2,16 g (0.01 Mol) Diacetylweinsäureanhydrid wird unter Schutzgas 2 Stunden auf 120°C erhitzt. Das glasartige Rohprodukt kann durch Säulenchromatographie (Kieselgel; Toluol/Aceton 8/2) gereinigt werden.

UV-Spektrum ($10^{-5}$M; Ethanol): $\lambda_{max}$ = 328 nm $\varepsilon_{max}$ = 17 400 $M^{-1}cm^{-1}$

Beispiel 6: 2-[3-(4-Dodecyloxy-phenyl)-acryloyloxy]-bernsteinsäure-1-dodecylester

(106)

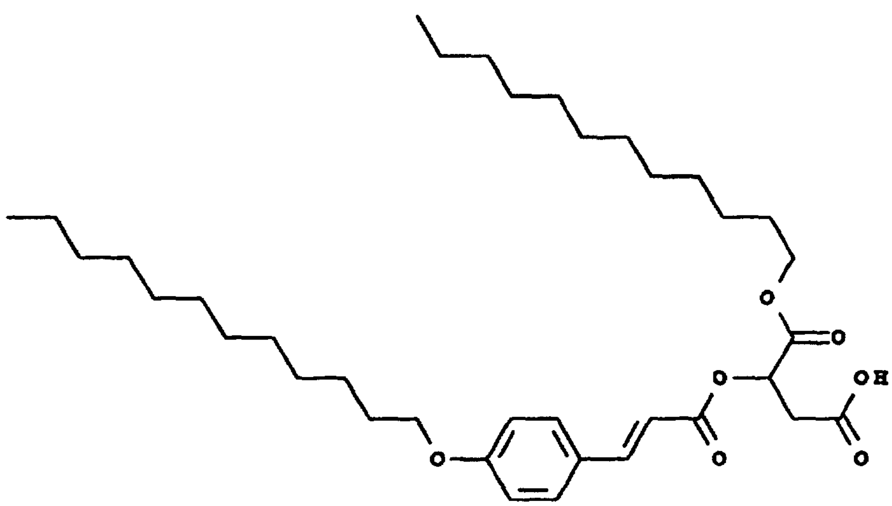

Die Herstellung von 4-Dodecyloxy-zimtsäurechlorid erfolgt wie in Beispiel 1 beschrieben.

30,9 g (0.088 Mol) Säurechlorid werden zusammen mit 5,36 g (0.04 Mol) DL-Aepfelsäure und 100 ml Chlorbenzol vorgelegt und unter Inertgas auf 130°C erhitzt. Nach 2 Stunden Rühren ist die HCl-Entwicklung beendet. Man tropft nun 7,7g (0.04 Mol) 1-Dodecanol, gelöst in 10 ml Chlorbenzol, zu und rührt weitere 3 Stunden am Rückfluss. Nach dem Erkalten wird der Niederschlag abgesaugt, mit Toluol und Hexan gewaschen und bei 80°C im Vakuum getrocknet. Das Rohprodukt kann durch Säulenchromatographie gereinigt werden (Kieselgel, Toluol/Aceton 80/20). Die Ausbeute beträgt 15,2g (62% d.Th).

UV-Spektrum ($10^{-5}$M; Ethanol): $\lambda_{max}$ = 311 nm $\varepsilon_{max}$ = 27 300 $M^{-1}cm^{-1}$

Beispiel 7: Bis-{2-[3-(4-dodecyloxy-phenyl)-acryloyloxy]-ethyl}-amino-essigsäure

(107)

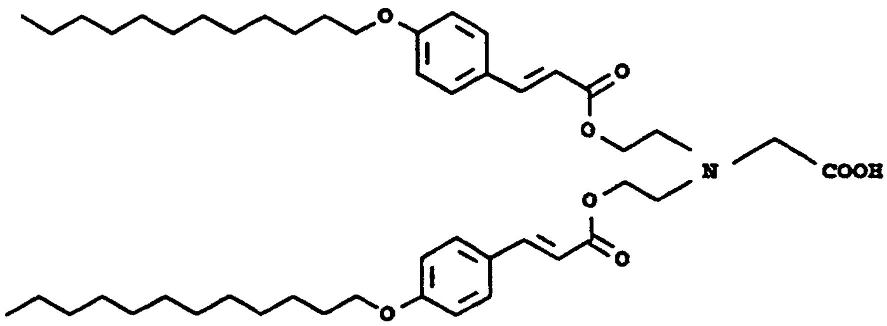

26,7g (0.075 Mol) 4-Dodecyloxy-zimtsäurechlorid (Darstellung siehe Beispiel 1.) und 4,9g (0.03 Mol) N, N-Bis-(hydroxyethyl)-glycin werden gut vermischt und unter Inertgas langsam auf 130-140°C erhitzt. Oberhalb 80°C wird der Ansatz rührbar und es tritt HCl-Entwicklung ein. Nach 4 Stunden ist die Reaktion beendet. Nach dem Abkühlen werden 100 ml Aceton/Wasser (95/5) zugegeben und der Ansatz wird 2 Stunden unter Rückfluss erhitzt. Man dampft zur Trockene ein und reinigt durch Säulenchromatographie (Kieselgel, Toluol/Aceton 80/20). Die Ausbeute des stark hygroskopischen Produktes beträgt 3,1g (13% d.Th).

UV-Spektrum ($10^{-5}$M; Ethanol): $\lambda_{max}$ = 313 nm $\varepsilon_{max}$ = 29 300 $M^{-1}cm^{-1}$

Beispiel 8: 2-Dodec-2-enyl-bernsteinsäure-1-{2-[3-(4-methoxy-phenyl)-acryloyloxy]-ethyl}-ester (Triethanolamin-Salz)

(108)

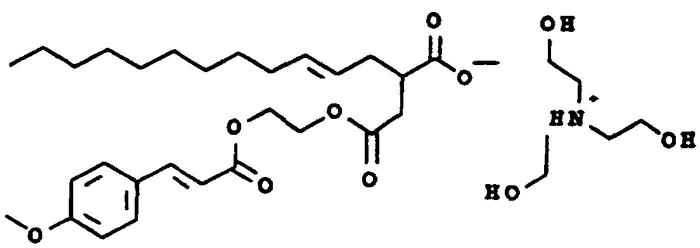

4-Methoxy-zimtsäure-(2-hydroxy-ethyl)-ester wird nach literaturbekannten Methoden (Veresterung) hergestellt.

9,0g (0.04 Mol) 4-Methoxy-zimtsäure-(2-hydroxy-ethyl)-ester und 10,6g (0.04 Mol) 2-Dodecenyl-bernsteinsäureanhydrid werden in 50 ml Toluol gelöst und bei 100-110°C gerührt. Nach 6 Stunden zeigt das Dünnschichtchromatogramm nahezu quantitativen Umsatz. Der Ansatz wird auf 55-60°C abgekühlt und mit 5,35g (0.036 Mol) Triethanolamin versetzt. Man rührt 6 Stunden nach und entfernt das Lösungsmittel im Vakuum. Man erhält 24,9 g (97,7% d. Th.) des leicht gefärbten, hochviskosen Produktes.

UV-Spektrum ($10^{-5}$M; Ethanol): $\lambda_{max}$ = 312 nm $\varepsilon_{max}$ = 26 400 $M^{-1}cm^{-1}$

Beispiel 9: 2-Dodec-2-enyl-bernsteinsäure-1-{[1-hydroxy-2-(2-benzotriazolyl)-6-t-butyl-4-(4-hydroxy-butyl)]-phenyl}-ester

(109)

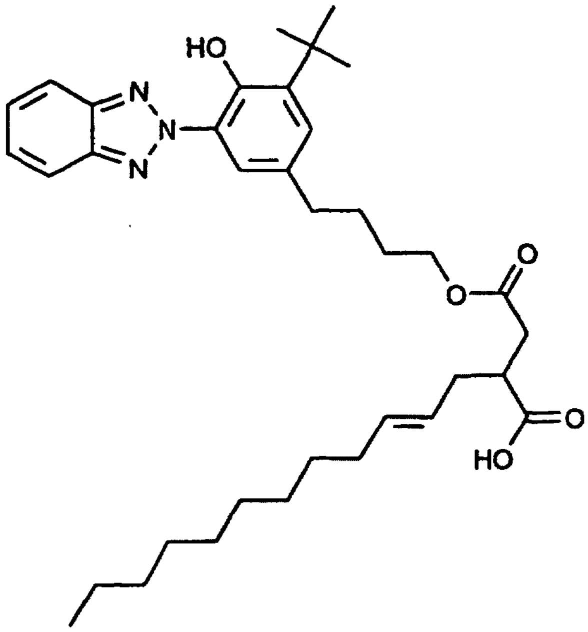

2-Benzotriazol-2-yl-6-t-butyl-4-(4-hydroxy-butyl)-phenol wird nach literaturbekannten Methoden hergestellt.

8,5g (0,025Mol) 2-Benzotriazol-2-yl-6-t-butyl-4-(4-hydroxy-butyl)-phenol und 6,9g (0,025 Mol) 2-Dodecenyl-bernsteinsäureanhydrid werden in 100ml Toluol gelöst und bei 100-110°C unter Stickstoff gerührt. Nach 18 Stunden zeigt das Dünnschichtchromatogramm nahezu quantitativen Umsatz. Man entfernt das Lösungsmittel im Vakuum und reinigt durch Säulenchromatographie (Toluol/Essigester=9/1; Kieselgel 40x5cm). Man erhält 5,4g eines leicht gefärbten, hochviskosen Produktes (36% d.Th.).

UV-Spektrum ($10^{-5}$M; Ethanol): $I_{max}$ = 340nm $I_{max}$ = 14 500 $M^{-1}cm^{-1}$

Anwendungsbeispiele:

Beispiel 11:

a. Herstellung von Liposomen

1-5 g der Verbindung der Formel (101a) (Herstellung vgl. Beispiel 1) werden in 100 ml N-Methyl-Pyrrolidon (NMP) gelöst. Anschließend wird diese Lösung tropfenweise zu 900 ml 0,9%iger wässeriger NaCl-Lösung gegeben oder mit einer Spritze injiziert, wobei sich Liposomen bilden. Das Volumen der resultierenden Liposomen-Suspension wird mit Hilfe einer Diafiltrationsapparatur (Ultrasette, Skan AG, Basel) auf 50-100 ml eingeengt, so daß die Liposomen entsprechend auf 1-10% aufkonzentriert werden. Das Lösungsmittel wird dann gleichfalls mit Hilfe der Diafiltrationsapparatur ausgetauscht, indem weiter filtriert und das Volumen des abgetrennten Filtrats gleichzeitig durch reine 0,9%ige NaCl-Lösung ersetzt wird. Zum Lösungsmittelaustausch wird, bezogen auf die eingeengte Liposomen-Suspension, mindestens das fünffache Volumen an reiner 0,9%iger NaCl-Lösung verwendet. Dann verbleiben bei einem anfänglichen NMP-Gehalt von 10% noch maximal 0,07% NMP in der Suspension. Der Restgehalt an NMP läßt sich durch Erhöhung des Volumens der zum Austausch verwendeten 0,9%igen NaCl-Lösung beliebig verringern.

Alternativ werden Liposomen hergestellt, indem 1-5 g der Verbindung der Formel (101a) zunächst wiederum in 100 ml Lösungsmittel (NMP, Ethanol oder Diethylether) gelöst werden. Die Lösung wird dann in einen Rundkolben gegeben, erhitzt und das Lösungsmittel mit einem Rotationsverdampfer unter Vakuum abgezogen. Zum resultierenden Film auf der Innenwand des Kolbens werden nun 50-100 ml 0,9%ige NaCl-Lösung gegeben und dann geschüttelt. Die dabei entstehenden Liposomen werden anschließend für 10-60 Minuten mit Hilfe eines Ultraschallstabs beschallt (Branson Model 250 Sonifier, Skan AG, Basel), was zur Verkleinerung der Liposomen führt.

b. Bestimmung des Durchmessers der Liposomen

Der Durchmesser der Liposomen wird mit Hilfe der Photonenkorrelationsspektroskopie (ALV / Monomode-Faser Kompakt Goniometer System, ALV-Laser GmbH, Langen) bestimmt. Dazu wird die Liposomen-Suspension auf 0,02% bis 0,1% verdünnt und die Autokorrelationsfunktion der Streulichtfluktuationen bei vier Streuwinkeln (30°, 60°, 90° und 120°) gemessen. Mit Hilfe der CONTIN-Software erhält man daraus mittlere Diffusionskoeffizienten, die im Falle einer Winkelabhängigkeit auf den Streuwinkel 0° extrapoliert werden. Aus dem resultierenden Diffusionskoeffizienten ergibt sich mit der Stokes-Einstein-Beziehung der Durchmesser der Liposomen. Für die mit der Injektionsmethode hergestellten Liposomen der Verbindung der Formel (101a) wird gefunden:

$$d = (220 \pm 40)\ \text{nm}.$$

Dies gilt als Beweis, daß Liposomen vorliegen. Bei der zweiten Methode zur Liposomen-Präparation, die unter a. beschrieben ist, läßt sich der Durchmesser durch Variation der Beschallungszeit (10 bis 60 Minuten) zwischen 1000 und 200 nm einstellen.

c. Spektrale Eigenschaften:

Die maximale Extinktion der Verbindung der Formel (101a) liegt bei einer Wellenlänge von 310 nm. Bei dieser Wellenlänge beträgt die Extinktion einer 1%igen Lösung bei 1 cm optischer Schichtdicke:

$$E(1\%,1\text{cm}) = 612.$$

Die Halbwertsbreite der Absorptionsbande beträgt 58 nm.

d. Bestimmung von Lichtschutzfaktoren:

Lichtschutzfaktoren (LSF) werden nach der Methode von Diffey und Robson (J. Soc. Cosmet. Chem. 40 (1989) 127) bestimmt. Es werden also auf Transpore®-Band (3M) pro $cm^2$ 2µl der Formulierung aufgetragen. Die diffuse Transmission (Messung mit Integrationskugel) wird dann im spektralen Bereich zwischen 290 und 400 nm gegen unbeschichtetes Transpore®-Band gemessen und für die Berechnung des Sonnenschutzfaktors mit dem Empfindlichkeitsspektrum der Haut und dem Intensitätsspektrum der Sonne gewichtet. Es werden jeweils neun Messungen durchgeführt, wobei der höchste und der niedrigste Wert der resultierenden Sonnenschutzfaktoren nicht berücksichtigt werden, so daß die Mittelwertbildung schließlich mit sieben Meßwerten geschieht. Für eine 5%ige Liposomen-Suspension der Verbindung der Formel (101a) ergibt sich damit:

$$LSF = 8{,}6 \pm 1{,}7.$$

Beispiel 12:

a. Herstellung von Liposomen

Die Herstellung der Liposomen erfolgt wie in Beispiel 11a. beschrieben, jedoch wird an Stelle der Verbindung der Formel (101a) die Verbindung der Formel (101b) und anstelle von NMP als Lösungsmittel für die Injektion auf 65°C erwärmtes Ethanol verwendet.

b. Bestimmung des Durchmessers der Liposomen

Die Bestimmung des Durchmessers der Liposomen erfolgt wie in Beispiel 11 b. beschrieben. Für Liposomen der Verbindung der Formel (101 b) erhält man einen Wert von:

$$d = (190 \pm 30)\ \text{nm}.$$

c. Spektrale Eigenschaften

Die maximale Extinktion der Verbindung der Formel (101b) liegt bei einer Wellenlänge von 310 nm. Bei dieser Wellenlänge beträgt die Extinktion einer 1%igen Lösung bei 1 cm optischer Schichtdicke:

$$E(1\%,1cm) = 601.$$

Die Halbwertsbreite der Absorptionsbande beträgt 58 nm.

d. Bestimmung von Lichtschutzfaktoren

Die Bestimmung des Lichtschutzfaktors erfolgt analog wie in Beispiel 11e für die Verbindung der Formel (101a) beschrieben. Es ergibt sich mit einer 5%igen Suspension der Verbindung der Formel (101b) ein Wert von

$$LSF = 8,3 \pm 1,6.$$

Beispiel 13:

a. Herstellung von Liposomen

Die Herstellung der Liposomen erfolgt analog wie in Beispiel 11a. beschrieben, mit dem Unterschied, dass anstelle der Verbindung der Formel (101a) die Verbindung der Formel (102a) verwendet wird (Herstellung der Verbindung vgl. Beispiel 2). Als Lösungsmittel für die Injektion wird Ethanol oder NMP verwendet.

b. Bestimmung des Durchmessers der Liposomen

Die Bestimmung des Durchmessers der Liposomen erfolgt wie in Beispiel 11b. beschrieben. Für Liposomen der Verbindung der Formel (102a) erhält man einen Wert von:

$$d = (250 \pm 40)\ nm.$$

c. Spektrale Eigenschaften

Die maximale Extinktion der Verbindung der Formel (102a) liegt bei einer Wellenlänge von 328 nm. Bei dieser Wellenlänge beträgt die Extinktion einer 1%igen Lösung bei 1 cm optischer Schichtdicke:

$$E(1\%,1cm) = 273.$$

Die Halbwertsbreite der Absorptionsbande beträgt 71 nm.

d. Bestimmung von Lichtschutzfaktoren

Die Bestimmung des Lichtschutzfaktors erfolgt analog wie in Beispiel 11e. beschrieben. Es ergibt sich mit einer 5%igen Suspension der Verbindung der Formel (102a) ein Wert von

$$LSF = 3,0 \pm 0,3.$$

Beispiel 14:

a. Herstellung von Liposomen:

Die Herstellung der Liposomen erfolgt wie in Beispiel 11a. beschrieben, mit dem Unterschied, dass anstelle der Verbindung der Formel (101a) die Verbindung der Formel (102b) verwendet wird. Als Lösungsmittel für die Injektion

wird Ethanol oder NMP verwendet.

b. Bestimmung des Durchmessers der Liposomen

Die Bestimmung des Durchmessers der Liposomen erfolgt wie in Beispiel 11b. beschrieben. Für Liposomen der Formel (102b) ergibt sich ein Wert von:

$$d = (150 \pm 25)\ \text{nm}.$$

c. Spektrale Eigenschaften:

Die maximale Extinktion der Verbindung der Formel (102b) liegt bei einer Wellenlänge von 328 nm. Bei dieser Wellenlänge beträgt die Extinktion einer 1 %igen Lösung bei 1 cm optischer Schichtdicke:

$$E(1\%,1\text{cm}) = 274.$$

Die Halbwertsbreite der Absorptionsbande beträgt 69 nm.

d. Bestimmung von Lichtschutzfaktoren:

Die Bestimmung des Lichtschutzfaktors erfolgte analog wie in Beispiel 11 e. beschrieben. Es ergibt sich mit einer 5%igen Suspension der Verbindung der Formel (102b) ein Wert von

$$LSF = 3{,}0 \pm 0{,}3.$$

Beispiel 15:

a. Herstellung von Liposomen

Die Herstellung der Liposomen erfolgt wie in Beispiel 11a. beschrieben, mit dem Unterschied, dass anstelle der Verbindung der Formel (101a) die Verbindung der Formel (104) verwendet wird (Herstellung der Verbindung vgl. Beispiel 4). Als Lösungsmittel für die Injektion wird Ethanol oder NMP verwendet.

b. Bestimmung des Durchmessers der Liposomen

Die Bestimmung des Durchmessers der Liposomen erfolgt wie in Beispiel 11b. beschrieben. Für Liposomen der Verbindung der Formel (104) ergibt sich ein Wert von:

$$d = (120 \pm 15)\ \text{nm}.$$

c. Spektrale Eigenschaften

Die maximale Extinktion der Verbindung der Formel (104) liegt bei einer Wellenlänge von 303 nm. Bei dieser Wellenlänge beträgt die Extinktion einer 1%igen Lösung bei 1 cm optischer Schichtdicke:

$$E(1\%,1\text{cm}) = 654.$$

Die Halbwertsbreite der Absorptionsbande beträgt 39 nm.

d. Bestimmung von Lichtschutzfaktoren

Die Bestimmung des Lichtschutzfaktors erfolgt wie in Beispiel 11b. beschrieben. Es ergibt sich mit einer 5%igen Suspension der Verbindung der Formel (104) ein Wert von

$$LSF = 5,0 \pm 0,5.$$

Beispiel 16:

a. Herstellung von Liposomen:

Die Herstellung der Liposomen erfolgt wie in Beispiel 11a. beschrieben, mit dem Unterschied, dass man anstelle der Verbindung der Formel (101a) die Verbindung der Formel (105) verwendet (Herstellung der Verbindung vgl. Beispiel 5). Als Lösungsmittel für die Injektion wird Ethanol oder NMP verwendet.

b. Bestimmung des Durchmessers der Liposomen

Die Bestimmung des Durchmessers der Liposomen erfolgt wie in Beispiel 11b beschrieben. Für Liposomen der Formel (105) ergibt sich einen Wert von:

$$d = (150 \pm 25)\ nm.$$

c. Spektrale Eigenschaften

Die maximale Extinktion der Verbindung der Formel (105) liegt bei einer Wellenlänge von 328 nm. Bei dieser Wellenlänge beträgt die Extinktion einer 1%igen Lösung bei 1 cm optischer Schichtdicke:

$$E(1\%,1cm) = 215.$$

Die Halbwertsbreite der Absorptionsbande beträgt 68 nm.

d. Bestimmung von Lichtschutzfaktoren

Die Bestimmung des Lichtschutzfaktors erfolgt wie in Beispiel 11e. beschrieben. Es ergibt sich mit einer 5%igen Suspension der Verbindung der Formel (105) ein Wert von

$$LSF = 2,3 \pm 0,3.$$

Beispiel 17:

a. Herstellung von Liposomen:

Die Herstellung der Liposomen erfolgt wie in Beispiel 11a. beschrieben, mit dem Unterschied, dass man anstelle der Verbindung der Formel (101a) die Verbindung der Formel (106) verwendet (Herstellung der Verbindung vgl. Beispiel 6). Als Lösungsmittel für die Injektion wird Ethanol oder NMP verwendet.

b. Bestimmung des Durchmessers der Liposomen:

Die Bestimmung des Durchmessers der Liposomen erfolgt wie in Beispiel 11b. beschrieben. Für Liposomen der Formel (106) ergibt sich ein Wert von:

$$d = (180 \pm 30)\ nm.$$

c. Spektrale Eigenschaften:

Die maximale Extinktion der Verbindung der Formel (106) liegt bei einer Wellenlänge von 312 nm. Bei dieser Wellenlänge beträgt die Extinktion einer 1%igen Lösung bei 1 cm optischer Schichtdicke:

$$E(1\%,1cm) = 451.$$

Die Halbwertsbreite der Absorptionsbande beträgt 50 nm.

d. Bestimmung von Lichtschutzfaktoren:

Die Bestimmung des Lichtschutzfaktors erfolgt wie in Beispiel 11e. beschrieben. Es ergibt sich mit einer 5%igen Suspension der Verbindung der Formel (106) ein Wert von

$$LSF = 5,6 \pm 0,7.$$

Beispiel 18:

a. Herstellung von Liposomen:

Die Herstellung der Liposomen erfolgt wie in Beispiel 11a. beschrieben, mit dem Unterschied, dass anstelle der Verbindung der Formel (101a) die Verbindung der Formel (107) verwendet wird (Herstellung der Verbindung vgl. Beispiel 7). Als Lösungsmittel für die Injektion wird Ethanol oder NMP verwendet.

b. Bestimmung des Durchmessers der Liposomen:

Die Bestimmung des Durchmessers der Liposomen erfolgt wie in Beispiel 11b. beschrieben. Für Liposomen der Formel (107) ergibt sich ein Wert von:

$$d = (980 \pm 130)\ nm.$$

c. Spektrale Eigenschaften:

Die maximale Extinktion der Verbindung der Formel (107) liegt bei einer Wellenlänge von 313 nm. Bei dieser Wellenlänge beträgt die Extinktion einer 1%igen Lösung bei 1 cm optischer Schichtdicke:

$$E(1\%,1cm) = 347.$$

Die Halbwertsbreite der Absorptionsbande beträgt 52 nm.

d. Bestimmung von Lichtschutzfaktoren:

Die Bestimmung des Lichtschutzfaktors erfolgt wie in Beispiel 11e. beschrieben. Es ergibt sich mit einer 5%igen Suspension der Verbindung der Formel (107) ein Wert von

$$LSF = 3,8 \pm 0,4.$$

Beispiel 19:

a. Herstellung von Liposomen:

Die Herstellung der Liposomen erfolgt wie in Beispiel 11a beschrieben, mit dem Unterschied, dass anstelle der Verbindung der Formel (101a) die Verbindung der Formel (108) verwendet wird (Herstellung der Verbindung vgl. Beispiel 8). Als Lösungsmittel für die Injektion wird Ethanol oder NMP verwendet.

b. Bestimmung des Durchmessers der Liposomen:

Die Bestimmung des Durchmessers der Liposomen erfolgt wie in Beispiel 11 b beschrieben. Für Liposomen

der Verbindung der Formel (108) ergibt sich ein Wert von:

$$d = (170 \pm 30)\ nm.$$

c. Spektrale Eigenschaften:

Die maximale Extinktion der Verbindung der Formel (108) liegt bei einer Wellenlänge von 310 nm. Bei dieser Wellenlänge beträgt die Extinktion einer 1 %igen Lösung bei 1 cm optischer Schichtdicke:

$$E(1\%,1cm) = 418.$$

Die Halbwertsbreite der Absorptionsbande beträgt 53 nm.

d. Bestimmung von Lichtschutzfaktoren:

Die Bestimmung des Lichtschutzfaktors erfolgt wie in Beispiel 11e. beschrieben. Es ergibt sich mit einer 5%igen Suspension der Verbindung der Formel (108) ein Wert von

$$LSF = 4,8 \pm 0,5.$$

Beispiel 20:

a. Herstellung von Liposomen:

Die Herstellung der Liposomen erfolgt wie in Beispiel 11a. beschrieben, mit dem Unterschied, dass anstelle der Verbindung der Formel (101a) die Verbindung der Formel (109) verwendet wird (Herstellung der Verbindung vgl. Beispiel 9). Als Lösungsmittel für die Injektion wird NMP verwendet.

b. Spektrale Eigenschaften:

Die maximale Extinktion der Verbindung der Formel (107) liegt bei einer Wellenlänge von 339 nm. Bei dieser Wellenlänge beträgt die Extinktion einer 1%igen Lösung bei 1 cm optischer Schichtdicke:

$$E(1\%,1cm) = 237.$$

c. Bestimmung von Lichtschutzfaktoren:

Die Bestimmung des Lichtschutzfaktors erfolgt wie in Beispiel 11e. beschrieben. Es ergibt sich mit einer 5%igen Suspension der Verbindung der Formel (109) ein Wert von LSF = 5,5.

Beispiel 21: Bestimmung der Affinität von UV-Absorbern auf Haaren durch Bestimmung des Ausziehgrades aus Formulierungen oder Lösungen

10 ml einer 10 mmolaren UV-Absorberlösung und 1g Haarsträhne werden in einem Glasgefäss definiert und reproduzierbar geschüttelt. Mittels Spektralphotometer wird die Konzentrationsabnahme des UV-Absorbers in der Testformulierung bzw. -Lösung nach 30 und 60 Minuten bestimmt (siehe Tabelle 1). Der so bestimmte Ausziehgrad stellt ein Mass für die Substantivität des UV-Absorbers bzw. der Formulierung, in der enthalten ist, dar.

| Tabelle 1: Bestimmung des Ausziehgrades | | | |
|---|---|---|---|
| Verbindung der Formel | Eingesetzter UV-Absorber | Ausziehgrad [%] | |
| | | nach 30 Minuten | nach 60 Minuten |
| (108) | 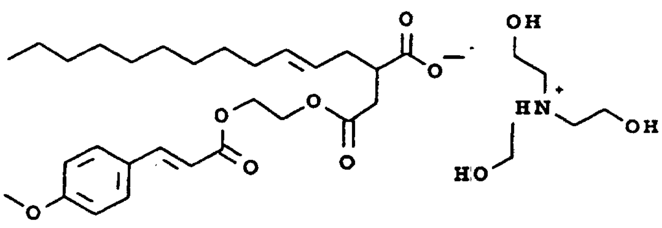 | 15,0 | 19,0 |

**Patentansprüche**

1. Verwendung von liposomogenen UV-Absorbern, enthaltend eine hydrophile Kopfgruppe (=Z), einen Abstandhalter (=W), einen UV-Chromophor (Q) mit einer Absorption im Bereich von 285 bis 400 nm und mindestens eine hydrophobe Schwanzgruppe (=A) entsprechend der Formel

(1) 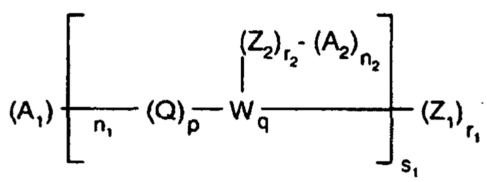

worin

| | |
|---|---|
| $A_1$ und $A_2$, | unabhängig voneinander, einen hydrophoben Rest; |
| Q | einen UV-Chromophor; |
| W | einen organischen Rest; |
| $Z_1$ und $Z_2$, | unabhängig voneinander, einen hydrophilen Rest; |
| $n_1$ und $n_2$, | unabhängig voneinander, eine Zahl von 0 bis 4, wobei $n_1=n_2=0$ nicht mitumfasst ist; |
| p | 1 oder 2; |
| q | eine Zahl von 0 bis 3; und |
| $r_1$ | 1 oder 2; |
| $r_2$ | 0 oder 1; und |
| $s_1$ | eine Zahl von 1 bis 3; |

bedeuten, zum Schützen von menschlichem Haar vor der schädigenden Einwirkung von UV-Strahlung.

2. Verwendung nach Anspruch 1, **dadurch gekennzeichnet, dass** der UV-Chromophor Q in Formel (1) ausgewählt ist aus der Klasse der

   ($Q_1$) Zimtsäureester; und
   ($Q_2$) Triazinderivate.

3. Verwendung nach Anspruch 1 oder 2, **dadurch gekennzeichnet, dass** der UV-Chromophor ($Q_1$) von Zimtsäuren

der Formel

(2) 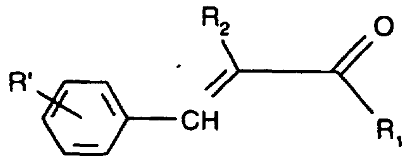

worin

R' Wasserstoff, $C_1$-$C_4$-Alkyl; oder $C_1$-$C_4$-Alkoxy;
$R_1$ $C_1$-$C_4$-Alkoxy; vorzugsweise Methoxy oder Ethoxy; und
$R_2$ Wasserstoff; $C_1$-$C_4$-Alkyl; oder -CN;

bedeuten, abgeleitet ist.

**4.** Verwendung nach einem der Ansprüche 1 bis 3, **dadurch gekennzeichnet, dass** der UV-Chromophor ($Q_2$) von einer $\alpha$-Hydroxyphenyl-s-triazinverbindung der Formel

(3) 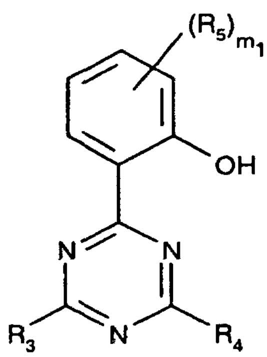

worin

$R_3$ und $R_4$, unabhängig voneinander, $C_1$-$C_5$-Alkyl; durch Hydroxy, $C_1$-$C_5$-Alkoxy, $C_1$-$C_5$-Alkylthio, Amino oder $C_1$-$C_5$-Mono- oder Dialkylamino substituiertes $C_1$-$C_{18}$-Alkyl; nicht substituiertes Phenyl; oder durch Chlor, Hydroxy, $C_1$-$C_{18}$-Alkyl und/oder $C_1$-$C_{18}$-Alkoxy substituiertes Phenyl;
$R_5$ $C_1$-$C_{18}$Alkyl; $C_1$-$C_{18}$Alkoxy; Halogen; Hydroxy; einen Rest der Formel

(3a) $-(O-CH_2-CH_2-)_t-R_{6;}$

oder einen Rest der Formel

(3b) 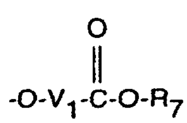

$R_7$ $C_1$-$C_5$-Alkyl oder $C_1$-$C_5$-Alkoxy-$C_1$-$C_5$-Alkyl;
$V_1$ einen $C_1$-$C_4$-Alkylenrest;
$m_1$ 0, 1 oder 2;
t 1 bis 5; und
$R_6$ Wasserstoff; oder $C_1$-$C_5$-Alkyl bedeuten,

abgeleitet ist.

5. Verwendung des eines haarkosmetischen Präparates, enthaltend mindestens 0,25 bis 5 Gew.%, bezogen auf das Gesamtgewicht der Zusammensetzung, eines liposomogenen UV-Absorbers der Formel (1) nach Anspruch 1 zum Schutz der Haare vor ultravioletter Strahlung, **dadurch gekennzeichnet, dass** es in Form eines Schampoo, einer Lotion, eines Gels oder einer Emulsion zur Spülung, vor oder nach dem Shampoonieren, vor oder nach einer Färbung oder Entfärbung, vor oder nach einer Dauerwelle oder einem Entfrisiervorgang, in Form einer Lotion, eines Schaums oder eines Gels zum Frisieren oder Behandeln, in Form einer Lotion, eines Schaums oder eines Gels zum Bürsten oder zu Wellengebung, in Form eines Haarlacks, in Form einer Zusammensetzung zur Dauerwelle oder zum Entfrisieren, zur Färbung oder Entfärbung der Haare vorliegt.

6. Verfahren zur Behandlung von Human-Haar zum Schützen vor der schädigenden Einwirkung von UV-Strahlung, **dadurch gekennzeichnet, dass** man das Haar mit einem Schampoo, einer Lotion, einem Gel oder einer Emulsion zur Spülung, vor oder nach dem Shampoonieren, vor oder nach einer Färbung oder Entfärbung, vor oder nach einer Dauerwelle oder einem Entfrisiervorgang, mit einer Lotion, einem Schaum oder einem Gel zum Frisieren, mit einer Lotion, einem Schaum oder einem Gel zum Bürsten oder zur Wellengebung, mit einem Haarlack, mit einer Zusammensetzung zur Dauerwelle oder zum Entfrisieren, zur Färbung oder Entfärbung der Haare behandelt, wobei das Schampoo, die Lotion, das Gel, die Emulsion, der Haarlack oder die Zusammensetzung zur Dauerwelle einen liposomogenen UV-Absorber der Formel (1) enthält.

## Claims

1. The use of a liposomogenic UV absorber comprising a hydrophilic head group (=Z), a spacer (=W), a UV chromophore (Q) having an absorption in the range from 285 to 400 nm and at least one hydrophobic tail group (=A), which has the formula

(1) 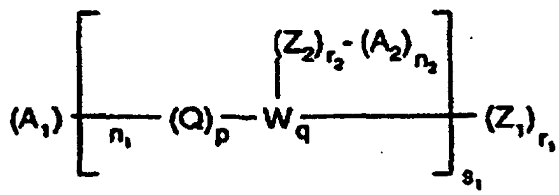

in which

| | |
|---|---|
| $A_1$ and $A_2$ | independently of one another are a hydrophobic radical; |
| Q | is a UV chromophore; |
| W | is an organic radical; |
| $Z_1$ and $Z_2$ | independently of one another are a hydrophilic radical; |
| $n_1$ and $n_2$ | independently of one another are a number from 0 to 4, where $n_1$=$n_2$=0 is not included; |
| p | is 1 or 2; |
| q | is a number from 0 to 3; |
| $r_1$ | is 1 or 2; |
| $r_2$ | is 0 or 1; and |
| $s_1$ | is a number from 1 to 3; |

for protecting human hair from the damaging effect of UV radiation.

2. The use according to claim 1, wherein the W chromophore Q in formula (1) is chosen from the dass consisting of

   ($Q_1$) cinnamic acid esters; and
   ($Q_2$) triazine derivatives.

3. The use according to claim 1 or 2, wherein the UV chromophore ($Q_1$) is derived from a cinnamic acid of the formula

(2) 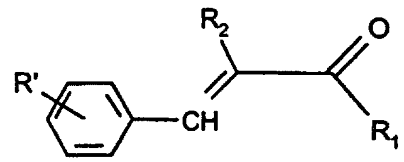

in which

R' is hydrogen, $C_1$-$C_4$alkyl; or $C_1$-$C_4$alkoxy;
$R_1$ is $C_1$-$C_4$alkoxy; preferably methoxy or ethoxy; and
$R_2$ is hydrogen; $C_1$-$C_4$alkyl; or -CN.

**4.** The use according to any one of claims 1 to 3, wherein the UV chromophore ($Q_2$) is derived from an $\alpha$-hydroxyphenyl-s-triazine compound of the formula

(3) 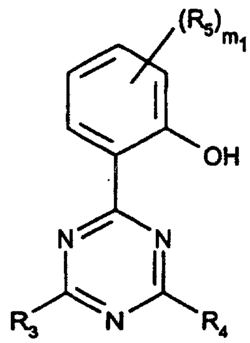

in which

$R_3$ and $R_4$ independently of one another are $C_1$-$C_5$alkyl; $C_1$-$C_{18}$alkyl which is substituted by hydroxyl, $C_1$-$C_5$alkoxy, $C_1$-$C_5$alkylthio, amino or $C_1$-$C_5$mono- or dialkylamino; unsubstituted phenyl; or phenyl which is substituted by chlorine, hydroxyl, $C_1$-$C_{18}$alkyl and/or $C_1$-$C_{18}$alkoxy;
$R_5$ is $C_1$-$C_{18}$alkyl; $C_1$-$C_{18}$alkoxy; halogen; hydroxyl; a radical of the formula

(3a) $-(O-CH_2-CH_2-)_t-R_6$;

or a radical of the formula

(3b) 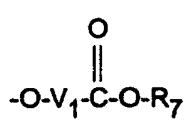 ;

$R_7$ is $C_1$-$C_5$alkyl or $C_1$-$C_5$alkoxy-$C_1$-$C_5$alkyl;
$V_1$ is a $C_1$-$C_4$alkylene radical;
$m_1$ is 0, 1 or 2;
t is 1 to 5; and
$R_6$ is hydrogen; or $C_1$-$C_5$alkyl.

**5.** The use of a hair cosmetics preparation comprising at least 0.25 to 5% by weight, based on the total weight of the composition, of a liposomogenic UV absorber of the formula (1) according to claim 1 for protecting the hair from ultraviolet radiation, which is in the form of a shampoo, a lotion, a gel or an emulsion for rinsing, before or after shampooing, before or after dyeing or bleaching, or before or after a permanent wave or a straightening operation,

in the form of a lotion, a foam or a gel for styling or treatment, in the form of a lotion, a foam or a gel for brushing or for waving, in the form of a hair lacquer, or in the form of a composition for a permanent wave or for straightening, or for dyeing or bleaching the hair.

**6.** A process for the treatment of human hair for protection from the damaging effect of UV radiation, which comprises treating the hair with a shampoo, a lotion, a gel or an emulsion for rinsing, before or after shampooing, before or after dyeing or bleaching, or before or after a permanent wave or a straightening operation, with a lotion, a foam or a gel for styling, with a lotion, a foam or a gel for brushing or for waving, with a hair lacquer or with a composition for a permanent wave or for straightening, or for dyeing or bleaching the hair, the shampoo, the lotion, the gel, the emulsion, the hair lacquer or the composition for a permanent wave comprising a liposomogenic UV absorber of the formula (1).

**Revendications**

**1.** Utilisation d'absorbeurs d'UV liposomogènes, contenant un groupe de tête hydrophile (= Z), un groupe espaceur (= W), un chromophore UV (Q) avec une absorption dans la plage de 285 à 400 nm et au moins un groupe de queue hydrophobe (= A) correspondant à la formule

(1) 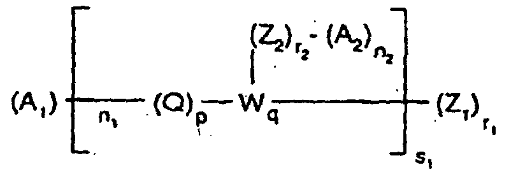

dans laquelle

$A_1$ et $A_2$ sont indépendamment l'un de l'autre un résidu hydrophobe ;
Q est un chromophore UV ;
W est un résidu organique ;
$Z_1$ et $Z_2$ sont indépendamment l'un de l'autre un résidu hydrophile ;
$n_1$ et $n_2$ sont indépendamment l'un de l'autre un nombre de 0 à 4, $n_1 = n_2 = 0$ n'étant pas inclus ;
p vaut 1 ou 2 ;
q un nombre valant de 0 à 3 ; et
$r_1$ vaut 1 ou 2 ;
$r_2$ vaut 0 ou 1 ; et
$s_1$ un nombre valant de 1 à 3 ;

pour protéger le cheveu humain contre les effets dommageables du rayonnement UV.

**2.** Utilisation selon la revendication 1, **caractérisée en ce que** le chromophore UV Q dans la formule (1) est choisi dans la classe

($Q_1$) des esters de l'acide cinnamique ; et
($Q_2$) des dérivés de triazine.

**3.** Utilisation selon la revendication 1 ou 2, **caractérisée en ce que** le chromophore UV ($Q_1$) est dérivé d'acides cinnamiques de formule

(2) 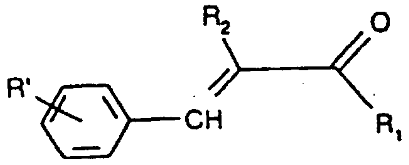

dans laquelle

R' est hydrogène, alkyle en $C_1$ à $C_4$ ou alcoxy en $C_1$ à $C_4$ ;
$R_1$ est alcoxy en $C_1$ à $C_4$, de préférence méthoxy ou éthoxy ; et
$R_2$ est hydrogène, alkyle en $C_1$ à $C_4$ ou -CN.

4. Utilisation selon l'une des revendications 1 à 3, **caractérisée en ce que** le chromophore UV ($Q_2$) est dérivé d'un composé d'α-hydroxyphényl-s-triazine de formule

(3) 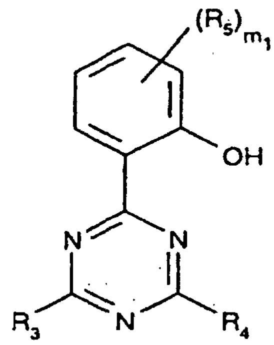

dans laquelle
$R_3$ et $R_4$ sont indépendamment l'un de l'autre alkyle en $C_1$ à $C_5$ ; alkyle en $C_1$ à $C_{18}$ substitué par hydroxy, alcoxy en $C_1$ à $C_5$, alkylthio en $C_1$ à $C_5$, amino ou mono- ou dialkylamino en $C_1$ à $C_5$ ; phényle non substitué ; ou phényle substitué par chloro, hydroxy, alkyle en $C_1$ à $C_{18}$ et/ou alcoxy en $C_1$ à $C_{18}$ ;
$R_5$ est alkyle en $C_1$ à $C_{18}$ ; alcoxy en $C_1$ à $C_{18}$ ; halogène ; hydroxy ; un résidu de la formule

(3a) $-(O-CH_2-CH_2-)_t-R_6$ ,

ou un résidu de formule

(3b) 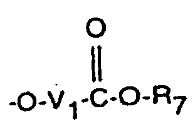

$R_7$ est alkyle en $C_1$ à $C_5$ ou ($C_2$-$C_5$-alcoxy)alkyle en $C_1$ à $C_5$ ;
$V_1$ est un résidu alkylène en $C_1$ à $C_4$,
$m_1$ vaut 0, 1 ou 2 ;
t vaut 1 à 5 ; et
$R_6$ est hydrogène ou alkyle en $C_1$ à $C_5$.

5. Utilisation d'une préparation de soin capillaire contenant au moins 0,25 à 5% en poids, ramené au poids total de la composition, d'un absorbeur d'UV liposomogène de formule (1) selon la revendication 1 pour protéger les cheveux contre le rayonnement ultraviolet, **caractérisée en ce qu'**elle se présente sous la forme d'un shampooing, d'une lotion, d'un gel ou d'une émulsion de rinçage avant ou après le shampooinage, avant ou après une coloration ou décoloration, avant ou après une permanente où un défrisage, sous la forme d'une lotion, d'une mousse ou d'un gel de coiffage ou de traitement, sous la forme d'une lotion, d'une mousse ou d'un gel de brossage ou d'ondulation, sous la forme d'une laque pour les cheveux, sous la forme d'une composition pour permanente ou pour défrisage, pour la coloration ou la décoloration des cheveux.

6. Procédé de traitement du cheveu humain pour le protéger contre les effets dommageables du rayonnement UV, **caractérisé en ce qu'**on traite le cheveu avec un shampooing, une lotion, un gel ou une émulsion de rinçage, avant ou après le shampooinage, avant ou après une coloration ou décoloration, avant ou après une permanente ou un défrisage, avec une lotion, une mousse ou un gel de coiffage, avec une lotion, une mousse ou un gel de brossage ou d'ondulation, avec une laque pour les cheveux, avec une composition pour permanente ou pour

défrisage, pour la coloration ou la décoloration des cheveux, dans lequel le shampooing, la lotion, le gel, l'émulsion, la laque pour les cheveux ou la composition pour permanente contient un absorbeur d'UV liposomogène de formule (1).